# EUROPEAN PATENT SPECIFICATION

(11) **EP 2 034 009 B1**
(45) Date of publication and mention of the grant of the patent: **15.01.2014**
(21) Application number: 08172864.4
(22) Date of filing: 07.02.2003
(51) Int. Cl.: C12N 5/0783, C12N 5/00, C12N 5/02, A01N 63/00, A61K 35/26, A61K 35/28, A61L 27/38, C07K 16/28

(54) **Compositions and methods for restoring immune responsiveness in patients with immunological defects basing on cd3/cd28 costimulation**
Zusammensetzungen und Verfahren zur Wiederherstellung der Immunreaktivität in Patienten mit immunologischen Defekten basierend auf CD3/CD28 costimulation
Compositions et procedes de restauration de reponse immunitaire chez des patients souffrant de deficiences immunologiques basant sur cd3/cd28 costimulation

(30) Priority: 08.02.2002 US 355391 P; 26.04.2002 US 375733 P
(43) Date of publication of application: 11.03.2009
(62) Divisional of application: 03737733.0
(73) Proprietor: Life Technologies Corporation, Carlsbad, CA 92008 (US)
(72) Inventor: Bonyhadi, Mark, Sammamish, WA 98075 (US); Berenson, Ronald J., Mercer Island, WA 98040 (US)
(74) Representative: Weber, Birgit

(56) References cited:
- WO-A-01/62895
- WO-A-01/89539
- WO-A-03/024989
- BONYHADI M ET AL: "Xcellerate: An autologous T cell immunotherapy approach for treating B-cell lymphocytic leukemia (B-CLL)" BLOOD, W.B.SAUNDERS COMPANY, ORLANDO, FL, US, vol. 96, no. 11 Part 1, 16 November 2000 (2000-11-16), page 837a, XP000926673 ISSN: 0006-4971
- HAMI L ET AL: "XCELLERATETM: A PLATFORM PROCESS FOR THE GMP MANUFACTURE OF ACTIVATED T CELLS FOR THE TREATMENT OF PATIENTS WITH CANCER AND IMMUNE DYSFUNCTION" BLOOD, W.B.SAUNDERS COMPANY, ORLANDO, FL, US, vol. 96, no. 11, PART 1, 16 November 2000 (2000-11-16), page 840A, XP001030571 ISSN: 0006-4971
- EIRAKU N ET AL: "Clonal expansion within CD4+ and CD8+ T cell subsets in human T lymphotropic virus type I-infected individuals." JOURNAL OF IMMUNOLOGY (BALTIMORE, MD. : 1950) 15 DEC 1998, vol. 161, no. 12, 15 December 1998 (1998-12-15), pages 6674-6680, XP002512716 ISSN: 0022-1767
- RACIOPPI L ET AL: "Defective dendritic cell maturation in a child with nucleotide excision repair deficiency and CD4 lymphopenia" CLINICAL AND EXPERIMENTAL IMMUNOLOGY, vol. 126, no. 3, December 2001 (2001-12), pages 511-518, XP002320746 ISSN: 0009-9104
- MONJI TATSUE ET AL: "Activated T cells and their culture supernatants mediate differentiation and maturation of monocyte-derived dendritic cells" BLOOD, vol. 98, no. 11 Part 1, 16 November 2001 (2001-11-16), page 231a, XP009045065 & 43RD ANNUAL MEETING OF THE AMERICAN SOCIETY OF HEMATOLOGY, PART 1; ORLANDO, FLORIDA, USA; DECEMBER 07-11, 2001 ISSN: 0006-4971
- KALAMASZ D ET AL: "STORAGE/SHIPMENT OF FRESHLY HARVESTED OR CRYOPRESERVED XCELLERATETMACTIVATED T CELLS FOR CLINICAL APPLICATIONS" BLOOD, W.B.SAUNDERS COMPANY, ORLANDO, FL, US, vol. 96, no. 11, PART 2, 16 November 2000 (2000-11-16), page 316B, XP001030572 ISSN: 0006-4971
- BONYHADI M L ET AL: "Expansion of Antigen-Specific CTL Using CD3/CD28 Paramagnetic Microbeads (Xcellate TM Beads). For Adoptive Cellular Therapy of Melanoma" BLOOD, W.B.SAUNDERS COMPANY, ORLANDO, FL, US, vol. 98, no. 11, 16 November 2001 (2001-11-16), pages 32B-33B, XP002982063 ISSN: 0006-4971
- SMITH S: "TECHNOLOGY EVALUATION: C242-DM1, IMMUNOGEN INC" CURRENT OPINION IN MOLECULAR THERAPEUTICS, CURRENT DRUGS, LONDON,, GB, vol. 3, no. 2, April 2001 (2001-04), pages 198-203, XP008011026 ISSN: 1464-8431
- HAMI R F ET AL: "Optimizing the efficiency, reproducibility and cost of the Xcellerate process for clinical delivery of activated T cells" BLOOD, W.B.SAUNDERS COMPANY, ORLANDO, FL, US, vol. 98, no. 11, PART 2, 2001, pages 336B-337B, XP002973702 ISSN: 0006-4971
- SHIBUYA TERRY Y ET AL: "Anti-CD3/anti-CD28 bead stimulation overcomes CD3 unresponsiveness in patients with head and neck squamous cell carcinoma" ARCHIVES OF OTOLARYNGOLOGY HEAD AND NECK SURGERY, vol. 126, no. 4, April 2000 (2000-04), pages 473-479, XP009045082 ISSN: 0886-4470
- S Parmar: "Ex vivo expanded umbilical cord blood T cells maintain naive phenotype and TCR diversity", Cytotherapy, vol. 8, no. 2, 1 January 2006 (2006-01-01) , pages 149-157, XP055055675,
- LONG S A ET AL: "Standardized analysis for the quantification of Vbeta CDR3 T-cell receptor diversity", JOURNAL OF IMMUNOLOGICAL METHODS, ELSEVIER SCIENCE PUBLISHERS B.V.,AMSTERDAM, NL, vol. 317, no. 1-2, 20 December 2006 (2006-12-20), pages 100-113, XP028017617, ISSN: 0022-1759, DOI: 10.1016/J.JIM.2006.09.015 [retrieved on 2006-12-20]

## Description

### BACKGROUND OF THE INVENTION

### Field of the Invention

The present invention relates to a method for normalising a population of T cells with respect to the repertoire of at least one Vβ Family gene expressed.

### Description of the Related Art

The ability of T cells to recognise the universe of antigens associated with various cancers or infectious organisms is conferred by its T cell antigen receptor (TCR), which is made up of both an α (alpha) chain and a β (beta) chain or a γ (gamma) and a δ (delta) chain. The proteins which make up these chains are encoded by DNA which employs a unique mechanism for generating the tremendous diversity of the TCR. The TCR α and β or γ and δ chains are linked by a disulfide bond (Janeway, Travers, Walport, Immunobiology. Fourth Ed., 148-159. Elsevier Science Ltd/Garland Publishing 1999). The α/β or γ/δ heterodimer complexes with the invariant CD3 chains at the cell membrane and this complex recognises specific antigenic peptides bound to the MHC molecules, or, in the case of γ/δ T cells, may recognise moieties independent of MHC restriction. The enormous diversity of TCR specificities is generated much like immunoglobulin diversity, through somatic gene rearrangement. The β chain genes contain over 50 variable (V), 2 diversity (D), over 10 joining (J) segments, and 2 constant region segments (C). The α chain genes contain about 70 V segments, and over 60 J segments but no D segments, as well as one C segment. During T cell development in the thymus, the D to J gene rearrangement of the β chain occurs, followed by the V gene segment rearrangement to the DJ. This functional VDJβ exon is transcribed and spliced to join to a Cβ. For the α chain, a Vα gene segment rearranges to a Jα gene segment to create the functional exon that is then transcribed and spliced to the Cα.

The ability of V, D, and J gene segments to combine together randomly introduces a large element of combinatorial diversity into the TCR repertoire. The precise point at which V, D, and J segments join can vary, giving rise to local amino acid diversity at the junction. The exact nucleotide position of joining can differ by as much as 10 residues resulting in deletion of nucleotides from the ends of the V, D, and J gene segments, thereby producing codon changes at the junctions of these segments. Diversity is further increased during the rearrangement process when additional nucleotides not encoded by either gene segment are added at the junction between the joined gene segments. (The variability created by this process is called "N-region diversity.") (Janeway, Travers, Walport. Immunobiology. Fourth Ed., 98 and 150. Elsevier Science Ltd/Garland Publishing. 1999).

The level of diversity for the T cell repertoire can be measured, in part, by evaluating which TCR Vβ chains are being employed by individual T cells within a pool of circulating T cells, and by the number of random nucleotides inserted next to the Vβ gene. In general, when the circulating T cell pool contains T cells expressing the full range of TCR Vβ chains and when those individual Vβ chains are derived from gene recombination events which utilize the broadest array of inserted nucleotides, the T cell arm of the immune system will have its greatest potential for recognizing the universe of potential antigens. When the range of TCR Vβ chains expressed by the circulating pool of T cells is limited or reduced, and when expressed TCRs utilize chains encoded by recombined genes with limited nucleotide insertions, the breadth of the immune response potential is correspondingly reduced. The consequences of this are a reduced ability to respond to the wide variety of antigens leading to increased risks of infection and cancer.

Spectratype analysis is a recently developed method for measuring TCR Vβ, Vα, Vγ, or Vδ gene usage by a pool of T cells and levels of nucleotide insertion during the recombination process in T cell development (as described in U.S. Patent No. 5,837,447). Spectratype analysis can be used to measure the breadth or narrowness of the T cell immune response potential.

Binding of αβ TCR to the antigenic peptide bound in the context of an MHC molecule on the antigen presenting cell (APC) is the central event in T-cell activation, which occurs at an immunological synapse at the point of contact between the T-cell and the APC. To sustain T-cell activation, T lymphocytes typically require a second co-stimulatory signal. Co-stimulatian is typically necessary for a T helper cell to produce sufficient cytokine levels that induce clonal expansion. Bretscher, Immunol. Today 13:74, 1992; June et al., Immunol. Today 15:321, 1994. The major co-stimulatory signal occurs when a member of the B7 family ligands (CD80 (B7.1) or CD86 (B7.2)) on an activated antigen-presenting cell (APC) binds to CD28 on a T-cell.

Methods of stimulating the expansion of certain subsets of T-cells have the potential to generate a variety of T-cell compositions useful in immunotherapy. Successful immunotherapy can be aided by increasing the polyclonality, reactivity, and quantity of T-cells by efficient stimulation.

The various techniques available for expanding human T-cells have relied primarily on the use of accessory cells and/or exogenous growth factors, such as interleukin-2 (IL-2). IL-2 has been used together with an anti-CD3 antibody to stimulate T-cell proliferation, predominantly expanding the CD8⁺ subpopulation of T-cells. Both APC signals are thought to be required for optimal T-cell activation, expansion, and long-term survival of the T-cells upon re-infusion. The requirement for MHC-matched APCs as accessory cells presents a significant problem for long-term culture systems because APCs are relatively short-lived. Therefore, in a long-term culture system, APCs must be continually obtained from a source and replenished. The necessity for a renewable supply of accessory cells is problematic for treatment of immunodeficiencies in which accessory cells are affected. In addition, when treating viral infection, if accessory cells carry the virus, the cells may contaminate the entire T-cell population during long-term culture.

Methods previously available in the art have made use of anti-CD3 and anti CD28 for the expansion of T-cells. However, none of these methods has described using such or similar methods to increase the polyclonality of a T cell population nor the beneficial results thereof. Furthermore, the applicability of expanded T-cells has been limited to only a few disease states. Moreover, the methods previously available tend to further skew the clonality of the T cell population rather than increase and/or maintain the polyclonality of a T cell population. For maximum *in vivo* effectiveness, theoretically, an *ex vivo*- or *in vivo*-generated, activated T-cell population should be in a state that can maximally orchestrate an immune response to cancer, infectious disease, or other disease states. The present invention provides methods to generate an increased number of more highly activated and more pure T-cells that have increased polyclonality in TCR expression.

### SUMMARY OF THE INVENTION

The scope of the present invention is defined by the claims and any information that does not fall within the claims is provided for information only.

The present invention provides an *ex vivo* method for normalising a population of T cells obtained from an individual with respect to the repertoire of at least one Vβ family gene expressed, wherein the at least one Vβ family gene is selected from the group consisting of Vβ 4, 9, 11, 13, 14, 15 and 22, said method comprising:
(a) providing a population of cells from an individual wherein at least a portion thereof comprises T cells;
(b) exposing the population of cells to a first and a second agent, wherein the first agent comprises an anti-CD3 antibody, or antigen-binding fragment thereof, and the second agent comprises an anti-CD28 antibody, or antigen-binding fragment thereof; wherein said first and second agents are attached to a surface;
and wherein the exposure of said T cells to said first or second agent is for a time sufficient to normalise with respect to expression of at least one Vβ family gene.

In the method of the invention, said first and second agents are preferably attached to said surface by covalent attachment, by direct attachment or by indirect attachment.

In a preferred embodiment of the present invention, the resulting normalised T cell population is formulated with one or more pharmaceutically or physiologically acceptable carriers, diluents or excipients.

In one embodiment of the present invention, the normalisation comprises a shift from monoclonality to oligoclonality, a shift from monoclonality to polyclonality, or a shift from oligoclonality to polyclonality, of the T cell population as measured by a Vβ, Vα, Vγ, and/or Vδ spectratype profile of at least one Vβ, Vα, Vγ, and/or Vδ family gene. In another embodiment of the methods provided herein, the shift comprises an increase in polyclonal T cells expressing at least one Vβ, Vα, Vγ, and/or Vδ family gene to sufficient members for use in therapy.

In the method of the invention, said first and second agents are preferably attached to said surface by covalent attachment, by direct attachment or by indirect attachment.

In a preferred embodiment of the present invention, the resulting normalised T cell population is formulated for the treatment of an immunocompromised individual suffering from a T cell population with a restricted repertoire of Vβ family gene expression. In certain embodiments, the polyclonality of the infused T cells is maintained *in vivo* for at least 3 to 6 months to a year following infusion.

In one aspect the immunocompromised individual has a cancer. The cancer may be one of melanoma, non-Hodgkin's lymphoma, Hodgkin's disease, leukemia, plasmocytoma, sarcoma, glioma, thymoma, breast cancer, prostate cancer, colon-rectal cancer, kidney cancer, renal cell carcinoma, pancreatic cancer, nasopharyngeal carcinoma, breast cancer, lung cancer, ovarian cancer, cervical cancer, multiple myeloma, heptocellular carcinoma, acute lymphoblastic leukemia (ALL), acute myelogenous leukemia (AML), chronic myleogenous leukemia (CML), large granular lymphocyte leukemia (LGL), and chronic lymphocytic leukemia (CLL). In one preferred embodiment, the cancer is B-cell lymphocytic leukemia.

In another aspect, the immunocompromised individual is infected with an infectious organism. The infectious organism may comprise a virus, such as a single stranded RNA virus or a single stranded DNA virus, human immunodeficiency virus (HIV), hepatitis A, B or C virus, herpes simplex virus (HSV), human papilloma virus (HPV), cytomegalovirus (CMV), Epstein-Barr virus (BBV), a parasite, a bacterium, *M. tuberculosis, Pneumocystis carinii, Candida,* or *Aspergillus* or a combination thereof.

In another aspect, the immunocompromised individual has a congenital genetic disorder such as severe combined immunodeficiency (SCID) or common variable immunodeficiency (CVID). In certain embodiments, the individual is immunocompromised as a result of treatment associated with cancer. In certain embodiments, the individual is immunocompromised as a result of treatment associated with hematopoeitic stem cell transplantation, bone marrow transplantation, cord blood, allogeneic, autologous, or xenogeneic cell transplantation, chemotherapy, radiation therapy, treatment with cytotoxic agents, treatment with an immunosuppressive agent (*e.g.,* cyclosporine, corticosteroid, and the like). In a further embodiment, the immunocompromised individual has an immunodeficiency or an autoimmune disease. In yet a further embodiment, the immunocompromised individual has a chronic disease affecting the kidney, liver, or the pancreas. In one particular embodiment, the individual has diabetes. In one embodiment, the immunocompromised individual is affected by old age. In a further embodiment, the immunocompromised individual has undergone gene therapy, or other procedure involving gene transduction that has resulted in a skewing of the T cell repertoire.

In another aspect, the immunocompromised individual is afflicted with a disorder associated with altered or skewed T cell repertoire, including but not limited to, diseases such as, rheumatoid arthritis, multiple sclerosis, insulin dependent diabetes, Addison's disease, celiac disease, chronic fatigue syndrome, inflammatory bowel disease, ulcerativecolitis, Crohn's disease, Fibromyalgia, systemic lupus erythematosus, psoriasis, Sjogren's syndrome, hyperthyroidism/Graves disease, hypothyroidism/Hashimoto's disease, Insulin-dependent diabetes (type 1), Myasthenia Gravis, endometriosis, scleroderma, pernicious anemia, Goodpasture syndrome, Wegener's disease, glomerulonephritis, aplastic anemia, any of a variety of cytopenias, paroxysmal nocturnal hemoglobinuria, myelodysplastic syndrome, idiopathic thrombocytopenic purpura, autoimmune hemolytic anemia, Fanconi anemia, Evan's syndrome, Factor VIII inhibitor syndrome, Factor IX inhibitor syndrome, systemic vasculitis, dermatomyositis, polymyositis and rheumatic fever. The methods and compositions described herein can be used to treat haematological disorders characterised by low blood counts.

In another aspect, the immunocompromised individual is afflicted with a neurological disorder associated with T cell repertoire skewing or cardiovascular disease.

One embodiment of the present invention provides for formalising a population of T cells in an immunocompromised individual wherein the immunocompromised individual is afflicted with an autoimmune disease. In certain embodiments, autoimmune disease includes, but is not limited to, rheumatoid arthritis, multiple sclerosis, insulin dependent diabetes, Addison's disease, celiac disease, chronic fatigue syndrome, inflammatory bowel disease, ulcerativecolitis, Crohn's disease, Fibtomylagia, systemic lupus erythematosus, psoriasis, Sjorgen's syndrome, hyperthyroidism/Graves disease, hypothyroidism/Hashiomoto's disease, Insulin-dependent diabetes (type 1), and Myasthenia Gravis. In a further embodiment, the immunocompromised individual has been treated with chemotherapy. In yet a further embodiment, the immunocompromised individual has been treated with a cytotioxic agent. In another aspect, the immunocompromised individual has been treated with an immunosuppressive agent. In one aspect, the immunocompromised individual is afflicted with a haematological disorder associated with cytopenia, including, but not limited to, aplastic anaemia, myclodisplastic syndrome, Fanconi anaemia, idiopathic thrombocytopenic purpura and autoimmune haemolytic anaemia.

The present invention may be useful where an individual has undergone T-cell ablative therapy using either chemotherapy agents such as, fludarabine, external-beam radiation therapy (XRT), cyclophosphamide, or antibodies such as OKT3 or CAWATH, or following B-cell ablative therapy such as agents that react with CD20, e.g., Rituxan. The dosage of the above treatments to be administered to a patient will vary with the precise nature of the condition being treated and the recipient of the treatment. The scaling of dosages for human administration can be performed according to art-accepted practices. The dose for CAMPATH, for example, will generally be in the range 1 to about 100 mg for an adult patient, usually administered daily for a period between 1 and 30 days. The preferred daily dose is 1 to 10 mg per day although in some instances larger doses of up to 40 mg per day may be used (described in U.S. Patent No. 6,120,766).

In yet a further embodiment, said first and second agents are attached to said surface or said second surface by covalent attachment. In other embodiments, said first and second agents are attached to said surface or said second surface by direct attachment or indirect attachment.

### BRIEF DESCRIPTION OF THE DRAWINGS

Figure 1 is a schematic representation of a TCR Vβ chain spectratype analysis, illustrating typical spectratype profiles of polyclonal, oligoclonal, and monoclonal T cell populations.
Figure 2 shows a comparison of T cell repertoire as measured by spectratype analysis for unactivated T cells, T cells activated with OKT3 and IL-2 and T cells activated using the XCELLERATE^{™} process.
Figure 3 is a spectratype analysis of T cells from a B-CLL patient before and after XCELLERATE^{™} activation and shows that the XCELLERATE^{™} process corrects the T cell deficit observed in the patient.
Figure 4 is a graph illustrating the total level of T cell repertoire "perturbation" for 8 donors using the Goroshov Perturbation Index (Gorochov, G., Neumann, A. U., Kereveur, A., Parizot, C., Li, T., Katlama, C., Karmochkine, M., Raguin, G., Autran, B., and Debre, P. Nat.Med, 4: 215-221, 1998.).
Figure 5a and 5b are bar graphs showing flow cytometric analysis of TCR Vβ cell surface expression for several Vβ families on unmanipulated CD4+ and CD8+ T cells from 2 normal donors, compared to unmanipulated and XCELLERATED^{™} CD4+ and CD8+ T cells from 2 B-CLL donors. The graphs show the percent of CD8 (5a) and CD4 (5b) cells expressing representative TCR Vβ family proteins on their surface.

Figure 6 is a line graph showing that the XCELLERATE^{™} process improves lymphocyte recovery in transplanted myeloma patients.

### DETAILED DESCRIPTION OF THE INVENTION

The scope of the present invention is defined by the claims and any information that does not fall within the claims is provided for information only.

Prior to setting forth the invention, it may be helpful to an understanding thereof to set forth definitions of certain terms that will be used hereinafter.

The term "biocompatible", as used herein, refers to the property of being predominantly non-toxic to living cells.

The term "stimulation", as used herein, refers to a primary response induced by ligation of a cell surface moiety. For example, in the context of receptors, such stimulation entails the ligation of a receptor and a subsequent signal transduction event. With respect to stimulation of a T cell, such stimulation refers to the ligation of a T cell surface moiety that in one embodiment subsequently induces a signal transduction event, such as binding the TCR/CD3 complex. Further, the stimulation event may activate a cell and up or downregulate expression of cell surface molecules such as receptors or adhesion molecules, or up or downregulate secretion of a molecule, such as downregulation of Tumor Growth Factor beta (TGF-β). Thus, ligation of cell surface moieties, even in the absence of a direct signal transduction event, may result in the reorganization of cytoskeletal structures, or in the coalescing of cell surface moieties, each of which could serve to enhance, modify, or alter subsequent cell responses.

The term "activation", as used herein, refers to the state of a cell following sufficient cell surface moiety ligation to induce a measurable morphological, phenotypic, and/or functional change. Within the context of T cells, such activation may be the state of a T cell that has been sufficiently stimulated to induce cellular proliferation. Activation of a T cell may also induce cytokine production and/or secretion, and up or downregulation of expression of cell surface molecules such as receptors or adhesion molecules, or up or downregulation of secretion of certain molecules, and performance of regulatory or cytolytic effector functions. Within the context of other cells, this term infers either up or down regulation of a particular physico-chemical process.

The term "target cell", as used herein, refers to any cell that is intended to be stimulated by cell surface moiety ligation.

An "antibody", as used herein, includes both polyclonal and monoclonal antibodies (mAb); primatized (*e.g.,* humanized); murine; mouse-human; mouse-primate; and chimeric; and may be an intact molecule, a fragment thereof (such as scFv, Fv, Fd, Fab, Fab' and F(ab)'₂ fragments), or multimers or aggregates of intact molecules and/or fragments; and may occur in nature or be produced, *e.g.,* by immunization, synthesis or genetic engineering; an "antibody fragment," as used herein, refers to fragments, derived from or related to an antibody, which bind antigen and which in some embodiments may be derivatized to exhibit structural features that facilitate clearance and uptake, *e.g.,* by the incorporation of galactose residues. This includes, *e.g.,* F(ab), F(ab)'₂, scFv, light chain variable region (V_{L}), heavy chain variable region (V_{H}), and combinations thereof.

The term "protein", as used herein, includes proteins, glycoproteins and other cell-derived modified proteins, polypeptides and peptides; and may be an intact molecule, a fragment thereof, or multimers or aggregates of intact molecules and/or fragments; and may occur in nature or be produced, *e.g.,* by synthesis (including chemical and/or enzymatic) or genetic engineering.

The term "agent", "ligand", or "agent that binds a cell surface moiety", as used herein, refers to a molecule that binds to a defined population of cells. The agent may bind any cell surface moiety, such as a receptor, an antigenic determinant, or other binding site present on the target cell population. The agent may be a protein, peptide, antibody and antibody fragments thereof, fusion proteins, synthetic molecule, an organic molecule (*e.g.,* a small molecule), or the like. Within the specification and in the context of T cell stimulation, antibodies are used as a prototypical example of such an agent

The term "cell surface moiety" as used herein may refer to a cell surface receptor, an antigenic determinant, or any other binding site present on a target cell population.

The terms "agent that binds a cell surface moiety" and "cell surface moiety", as used herein, should be viewed as a complementary/anti-complementary set of molecules that demonstrate specific binding, generally of relatively high affinity.

A "co-stimulatory signal", as used herein, refers to a signal, which in combination with a primary signal, such as TCR/CD3 ligation, leads to T cell proliferation and/or activation.

"Separation", as used herein, includes any means of substantially purifying one component from another (*e.g.,* by nitration, affinity, buoyant density, or magnetic attraction).

A "surface", as used herein, refers to any surface capable of having an agent attached thereto and includes, without limitation, metals, glass, plastics, copolymers, colloids, lipids, cell surfaces, and the like. Essentially any surface that is capable of retaining an agent bound or attached thereto.

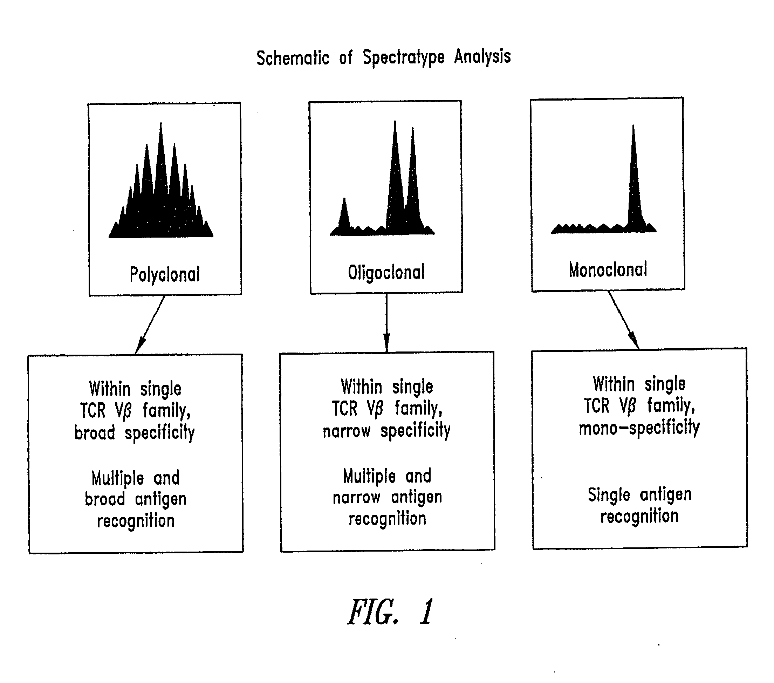

"Monoclonality", as used herein, in the context of a population of T cells, refers to a population of T cells that has a single specificity as defined by spectratype analysis (a measure of the TCR Vβ, Vα, Vγ, or Vδ chain hypervariable region repertoire). A population of T cells is considered monoclonal (or mono-specific) when the Vβ, Vα, Vγ, and/or Vδ spectratype profile for a given TCR Vβ, Vα, Vγ, and/or Vδ family has a single predominant peak (see Figure 1). Spectratype analysis distinguishes rearranged variable genes of a particular size, not sequence. Thus, it is understood that a single peak could represent a population of T cells expressing any one of a limited number of rearranged TCR variable genes (Vβ, Vα, Vγ, or Vδ) comprising any one of the 4 potential nucleotides (adenine (a), guanine (g), cytosine (c), or thymine (t)) or a combination of the 4 nucleotides at the junctional region. In certain embodiments of the present invention, it may be desirable to clone and sequence a particular band to determine the sequence(s) of the rearranged variable gene(s) present in the band representing a particular length..

"Oligoclonality", as used herein, in the context of a population of T cells, refers to a population of T cells that has multiple, but narrow antigen specificity as defined by spectratype analysis (a measure of the TCR β chain hypervariable region repertoire). A popular of T cells is considered oligoclonal when the Vβ spectratype profile for a given TCR Vβ family has between about 2 and 4 predominant peaks (see Figure 1).

"Polyclonality", as used herein, in the context of a population of T cells, refers to a population of T cells that has a multiple and broad antigen specificity as defined by spectratype analysis (a measure of the TCR β chain hypervariable region repertoire). A population of T cells is considered polyclonal when the Vβ spectratype profile for a given TCR Vβ family has multiple peaks, typically 5 or more predominant peaks and in most cases with Gaussian distribution (see for example Figures 1, 2 and 3).

"Restoring or increasing the polyclonality", or normalising of T cells, as used herein, refers to a shift from a monoclonal profile to an oligoclonal profile or to a polyclonal profile (in other words, a shift from monoclonality to oligoclonality or to polyclonality), or from an oligoclonal profile to a polyclonal profile (in other words, a shift from oligoclonality to polyclonality), in expressed TCR Vβ, Vα, Vγ, and/or Vα genes in a population of T cells, as measured by spectratype analysis or by similar analysis such as flow cytometry or sequence analysis. The shift from a monoclonal Vβ, Vα, Vγ and/or Vδ expression profile in a population of T cells to an oligoclonal profile or to a polyclonal profile is generally seen in at least one TCR Vβ, Vα, Vγ and/or Vδ family. In one aspect of the present disclosure, this shift is observed in 2, 3, 4 or 5 Vβ families. In certain embodiments of the present invention, a shift is observed in 6, 7, 8, 9 or 10 Vβ families. In a further embodiment of the present invention, a shift is observed in from 11, 12, 13 or 14 Vβ families. In a further embodiment of the present invention, a shift is observed in from 15 to 20 Vβ families. In a further embodiment of the present invention, a shift is observed in 10 to 24 Vβ families. In another embodiment, a shift is seen in all Vβ families. The functional significance of restoring or increasing the polyclonality of a population of T cells is that the immune potential, or the ability to respond to a full breadth of antigens, of the population of T cells is restored or increased. In certain aspects of the present invention, some T cells within a population may not have their TCRs engaged by the methods set forth herein (*e.g.,* T cells with downregulated TCR expression). However, by being in close proximity to T cells activated by the methods described herein, and the factors secreted by them, these T cells may in turn upregulate their TCR expression thereby resulting in a further increase in the polyclonality of the population ofT cells.

The term "animal" or "mammal" as used herein, encompasses all mammals, including humans. Preferably, the animal of the present invention is a human subject

The term "exposing" as used herein, refers to bringing into the state or condition of immediate proximity or direct contact.

The term "proliferation" as used herein, means to grow or multiply by producing new cells.

"Immune response or responsiveness" as used herein, refers to activation of cells of the immune system, including but not limited to, T cells, such that a particular effector function(s) of a particular cell is induced. Effector functions may include, but are not limited to, proliferation, secretion of cytokines, secretion of antibodies, expression of regulatory and/or adhesion molecules, and the ability to induce cytolysis.

"Stimulating an immune response" as used herein, refers to any stimulation such that activation and induction of effector functions of cells of the immune system are achieved.

"Immune response dysfunction" as used herein, refers to the inappropriate activation and/or proliferation, or lack thereof, of cells of the immune system, and/or the inappropriate secretion, or lack thereof, of cytokines, and/or the inappropriate or inadequate induction of other effector functions of cells of the immune system, such as expression of regulatory, adhesion, and/or homing receptors, and the induction of cytolysis.

The terms "preventing" or "inhibiting the development of a cancer or cancer cells" as used herein, means the occurrence of the cancer is prevented or the onset of the cancer is delayed.

The terms "treating or reducing the presence of a cancer or cancer cells" or "treating or reducing the presence of a tumor or tumor cells" as used herein, mean that the cancer or tumor growth is inhibited, which is reflected by, *e.g.,* tumor volume or numbers of malignant cells. The reduction of cancer can be determined using any number of techniques in the art including measurements of M-protein, PCR based assays, RNA and DNA hybridization assays, or *in situ* PCR or hybridization, *etc.* Tumor volume may be determined by various known procedures, *e.g.,* obtaining two dimensional measurements with a dial caliper.

"Preventing or inhibiting the development of an infectious disease" as used herein, means the occurrence of the infectious disease is prevented or the onset of the infectious disease is delayed, or the .spread of an existing infection is reversed or stabilized.

"Ameliorate" as used herein, is defined as: to make better; improve (The American Heritage College Dictionary, 3rd Edition, Houghton Mifflin Company, 2000).

"Particles" or "surface" as used herein, may include a colloidal particle, a microsphere, nanoparticle, a bead, or the like. A surface may be any surface capable of having a ligand bound thereto or integrated into, including cell surfaces (for example K562 cells), and that is biocompatible, that is, substantially non-toxic to the target cells to be stimulated. In the various embodiments, commercially available surfaces, such as beads or other particles, are useful (*e.g.,* Miltenyi Particles, Miltenyi Biotec, Germany; Sepharose beads, Pharmacia Fine Chemicals, Sweden; DYNABEADS™, Dynal Inc., New York; PURABEADS™, Prometic Biosciences, magnetic beads from Immunicon, Huntingdon Valley, PA, microspheres from Bangs Laboratories, Inc., Fishers, IN).

"Paramagnetic particles" as used herein, refer to particles, as defined above, that localize in response to a magnetic field.

The term "infectious disease" as used herein, refers to any disease that is caused by an infectious organism. Infectious organisms may comprise viruses, (*e.g.,* single stranded RNA viruses, single stranded DNA viruses, human immunodeficiency virus (HIV), hepatitis A, B, and C virus, herpes simplex virus (HSV), cytomegalovirus (CMV) Epstein-Barr virus (EBV), human papilloma virus (HPV)), parasites (*e.g*., protozoan and metazoan pathogens such as *Plasmodia* species, *Leishmania* species, *Schistosoma* species, *Trypanosoma* species), bacteria (*e.g., Mycobacteria*, in particular, *M. tuberculosis, Salmonella, Streptococci, E. coli, Staphylococci*), fungi (*e.g., Candida* species, *Aspergillus* species), *Pneumocystis carinii*, and prions (known prions infect animals to cause scrapie, a transmissible, degenerative disease of the nervous system of sheep and goats, as well as bovine spongiform encephalopathy (BSE), or "mad cow disease", and feline spongiform encephalopathy of cats. Four prion diseases known to affect humans are (1) kuru, (2) Creutzfeldt-Jakob Disease (CJD), (3) Gerstmann-Straussler-Scheinker Disease (GSS), and (4) fatal familial insomnia (FFI)). As used herein "prion" includes all forms of prions causing all or any of these diseases or others in any animals used--and in particular in humans and domesticated farm animals.

### STIMULATION, ACTIVATION, AND RESTORATION OF POLYCLONALITY OF T CELLS

The stimulated and activated T cells with increased polyclonality are generated by cell surface moiety ligation that induces activation. The stimulated and activated T cells with increased polyclonality are generated by activating a population of T cells and stimulating an accessory molecule on the surface of the T cells with a ligand which binds the accessory molecule, as described for example, in US patent application numbers 08/253,694, 08/435,816, 08/592,711, 09/183,055, 09/350,202, and 09/252,150, and patent numbers 6,352,694, 5,858,358 and 5,883,223.

Generally, T cell activation and restoration of polyclonality may be accomplished by cell surface moiety ligation, such as stimulating the T cell receptor (TCR)/CD3 complex or the CD2 surface protein. A number of anti-human CD3 monoclonal antibodies are commercially available, exemplary are, clone BC3 (XR-CD3; Fred Hutchinson Cancer Research Center, Seattle, WA), OKT3, prepared from hybridoma cells obtained from the American Type Culture Collection, and monoclonal antibody G19-4. Similarly, stimulatory forms of anti-CD2 antibodies are known and available. Stimulation through CD2 with anti-CD2 antibodies is typically accomplished using a combination of at least two different anti-CD2 antibodies. Stimulatory combinations of anti-CD2 antibodies that have been described include the following: the T11.3 antibody in combination with the T11.1 or T11.2 antibody (Meuer et al., Cell 36:897-906, 1984), and the 9.6 antibody (which recognizes the same epitope as T11.1) in combination with the 9-1 antibody (Yang et al., J. Immunol. 137:1097-1100, 1986). Other antibodies that bind to the same epitopes as any of the above described antibodies can also be used. Additional antibodies, or combinations of antibodies, can be prepared and identified by standard techniques. Stimulation may also be achieved through contact with superantigens (*e.g., Staphylococcus* enterotoxin A (SEA), *Staphylococcus* enterotoxin B (SEB), Toxic Shock Syndrome Toxin 1 (TSST-1)), endotoxin, or through a variety of mitogens, including but not limited to, phytohemagglutinin (PHA), phorbol myristate acetate (PMA) and ionomycin, lipopolysaccharide (LPS), T cell mitogen, and IL-2.

To further activate and increase polyclonality of a population of T cells, a co-stimulatory or accessory molecule on the surface of the T cells, such as CD28, is stimulated with a ligand that binds the accessory molecule. Accordingly, one of ordinary skill in the art will recognize that any agent, including an anti-CD28 antibody or fragment thereof capable of cross-linking the CD28 molecule, or a natural ligand for CD28 can be used to stimulate T cells. Exemplary anti-CD28 antibodies or fragments thereof useful in the context of the present invention include monoclonal antibody 9.3 (IgG2ₐ) (Bristol-Myers Squibb, Princeton, NJ), monoclonal antibody KOLT-2 (IgG1), 15E8 (IgG1), 248.23.2 (IgM), clone B-T3 (XR-CD28; Diaclone, Besançon, France) and EX5.3D10 (IgG2ₐ) (ATCC HB11373). Exemplary natural ligands include the B7 family of proteins, such as B7-1 (CD80) and B7-2 (CD86) (Freedman et al., J. Immunol. 137:3260-3267, 1987; Freeman et al., J. Immunol. 143:2714-2722, 1989; Freeman et al., J. Exp. Med. 174:625-631, 1991; Freeman et al., Science 262:909-911, 1993; Azuma et al., Nature 366:76-79, 1993; Freeman et al., J. Exp. Med. 178:2185-2192, 1993).

Other illustrative accessory molecules on the surface of the T cells that can be stimulated with a ligand that binds the accessory molecule in the present invention include, but are not limited to, CD54, Ox-40, LFA-1, ICOS, 41-BB, and CD40.

In addition, binding homologues of a natural ligand, whether native or synthesized by chemical or recombinant techniques, can also be used in accordance with the present invention. Other agents may include natural and synthetic ligands. Agents may include, but are not limited to, other antibodies or fragments thereof, growth factor, cytokine, chemokine, soluble receptor, steroid, hormone, mitogen, such as PHA, or other superantigens.

### EXPANSION OF T-CELL POPULATIONS

In one aspect of the present invention, *ex vivo* T-cell expansion can be performed by stimulation of a population of cells wherein at least a portion thereof comprises T cells. T-cells are stimulated with two or more agents, one that induces a primary signal and additional agents that induce one or more co-stimulatory signals. Ligands useful for stimulating a single signal or stimulating a primary signal and an accessory molecule that stimulates a second signal may be immobilized on a surface as described herein. A ligand or agent that is attached to a surface serves as a "surrogate" antigen presenting cell (APC). In the invention both primary and secondary agents are co-immobilized on a surface. In one embodiment, the molecule providing the primary activation signal, such as a CD3 ligand, and the co-stimulatory molecule, such as a CD28 ligand, are coupled to the same surface, for example, a particle. Further, as noted earlier, one, two, or more stimulatory molecules may be used on the same or differing surfaces.

Prior to expansion, a source of T-cells is obtained from a subject. The term "subject" is intended to include living organisms in which an immune response can be elicited (*e.g*., mammals). Examples of subjects include humans, dogs, cats, mice, rats, and transgenic species thereof. T cells can be obtained from a number of sources, including peripheral blood mononuclear cells, bone marrow, thymus, tissue biopsy, tumor, lymph node tissue, gut associated lymphoid tissue, mucosa associated lymphoid tissue, spleen tissue, or any other lymphoid tissue, and tumors. T cells can be obtained from T cell lines and from autologous or allogeneic sources. T cells may also be obtained from a xenogeneic source, for example, from mouse, rat, non-human primate, and pig. In certain embodiments of the present invention, T cells can be obtained from a unit of blood collected from a subject using any number of techniques known to the skilled artisan, such as ficoll separation. In one preferred embodiment, cells from the circulating blood of an individual are obtained by apheresis or leukapheresis. The apheresis product typically contains lymphocytes, including T-cells, monocytes, granulocytes, B cells, other nucleated white blood cells, red blood cells, and platelets. In one embodiment, the cells collected by apheresis may be washed to remove the plasma fraction and to place the cells in an appropriate buffer or media for subsequent processing steps. In one embodiment of the invention, the cells are washed with phosphate buffered saline (PBS). In an alternative embodiment, the wash solution lacks calcium and may lack magnesium or may lack many if not all divalent cations. As those of ordinary skill in the art would readily appreciate a washing step may be accomplished by methods known to those in the art, such as by using a semi-automated "flow-through" centrifuge (for example, the Cobe 2991 cell processor, Baxter) according to the manufacturer's instructions. After washing, the cells may be resuspended in a variety of biocompatible buffers, such as, for example, Ca-free, Mg-free PBS. Alternatively, the undesirable components of the apheresis sample may be removed and the cells directly resuspended in culture media.

In another embodiment, T-cells are isolated from peripheral blood lymphocytes by lysing or removing the red blood cells and depleting the monocytes, for example, by centrifugation through a PERCOLL™ gradient. A specific subpopulation of T-cells, such as CD28⁺, CD4⁺, CD8⁺, CD45RA⁺, and CD45RO⁺T-cells, can be further isolated by positive or negative selection techniques. For example, in one preferred embodiment, T-cells are isolated by incubation with anti-CD3/anti-CD28 (*i.e.,* 3x28)-conjugated beads, such as DYNABEADS® M-450 CD3/CD28 T, for a time period sufficient for positive selection of the desired T cells. In one embodiment, the time period is about 30 minutes. In a further embodiment, the time period ranges from 30 minutes to 36 hours or longer and all integer values there between. In a further embodiment, the time period is at least 1, 2, 3, 4, 5, or 6 hours. In yet another preferred embodiment, the time period is 10 to 24 hours. In one preferred embodiment, the incubation time period is 24 hours. For isolation of T cells from patients with leukemia, use of longer incubation times, such as 24 hours, can increase cell yield. Longer incubation times may be used to isolate T cells in any situation where there are few T cells as compared to other cell types, such in isolating tumor infiltrating lymphocytes (TIL) from tumor tissue or from immunocompromised individuals. Further, use of longer incubation times can increase the efficiency of capture of CD8+ T cells. For example, CD3⁺, CD28⁺ T cells can be positively selected using CD3/CD28 conjugated magnetic beads (*e.g*., DYNABEADS^{®} M-450 CD3/CD28 T Cell Expander). In one aspect of the present invention, enrichment of a T-cell population by negative selection can be accomplished with a combination of antibodies directed to surface markers unique to the negatively selected cells. A preferred method is cell sorting and/or selection via negative magnetic immunoadherence or flow cytometry that uses a cocktail of monoclonal antibodies directed to cell surface markers present on the cells negatively selected. For example, to enrich for CD4⁺ cells by negative selection, a monoclonal antibody cocktail typically includes antibodies to CD14, CD20, CD11b, CD16, HLA-DR, and CD8.

The present invention may use a T-cell population or composition that has been depleted or enriched for populations of cells expressing a variety of markers, such as CD62L, CD45RA or CD45RO, cytokines (*e.g*., IL-2, IFN-γ, IL-4, IL-10), cytokine receptors (*e.g*., CD25), perforin, adhesion molecules (*e.g*., VLA-1, VLA-2, VLA-4, LPAM-1, LFA-1), and/or homing molecules (*e.g*., L-Selectin), prior to expansion. Cells expressing any of these markers are depleted or positively selected by antibodies or other ligands/binding agents directed to the marker. One of ordinary skill in the art would readily be able to identify a variety of particular methodologies for depleting or positively selecting for a sample of cells expressing a desired marker.

With respect to monocyte depletion noted above, monocyte populations (*i.e.,* CD14⁺ cells) may be depleted from blood preparations prior to *ex vivo* expansion by a variety of methodologies, including anti-CD14 coated beads or columns, or utilization of the phagocytotic activity of these cells to facilitate removal or through adherence to plastic. Accordingly, in one embodiment, the invention uses paramagnetic particles of a size sufficient to be engulfed by phagocytotic monocytes. In certain embodiments, the paramagnetic particles are commercially available beads, for example, those produced by Dynal AS under the trade name Dynabeads™. Exemplary Dynabeads™ in this regard are M-280, M-450, and M-500. In one aspect, other non-specific cells are removed by coating the paramagnetic particles with "irrelevant" proteins (*e.g.,* serum proteins or antibodies). Irrelevant proteins and antibodies include those proteins and antibodies or fragments thereof that do not specifically target the T-cells to be expanded. In certain embodiments the irrelevant beads include beads coated with sheep anti-mouse antibodies, goat anti-mouse antibodies, and human serum albumin.

In brief such depletion of monocytes is performed by preincubating PBMC that have been isolated from whole blood using Ficoll, or apheresed peripheral blood with one or more varieties of irrelevant or non-antibody coupled paramagnetic particles at any amount that allows for removal of monocytes (approximately a 20:1 bead:cell ratio)for about 30 minutes to 2 hours at 22 to 37 degrees C, followed by magnetic removal of cells which have attached to or engulfed the paramagnetic particles. Preincubation can also be done at temperatures as low as 3-4 degrees C. Such separation can be performed using standard methods available in the art. For example, any magnetic separation methodology may be used including a variety of which are commercially available, (*e.g.,* DYNAL^{®} Magnetic Particle Concentrator (DYNAL MPC^{®})). Assurance of requisite depletion can be monitored by a variety of methodologies known to those of ordinary skill in the art, including flow cytometric analysis of CD14 positive cells, before and after said depletion.

T-cells for stimulation may also be frozen after the washing step, which does not require the monocyte-removal step. Wishing not to be bound by theory, the freeze and subsequent thaw step provides a more uniform product by removing granulocytes and to some extent monocytes in the cell population. After the washing step that removes plasma and platelets, the cells may be suspended in a freezing solution. While many freezing solutions and parameters are known in the art and will be useful in this context, one method involves using PBS containing 20% DMSO and 8% human serum albumin, or other suitable cell freezing media, the cells then are frozen to -80°C at a rate of 1° per minute and stored in the vapor phase of a liquid nitrogen storage tank.

The cell population may be stimulated as described herein, such as by contact with an anti-CD3 antibody or an anti-CD2 antibody immobilized on a surface, or by contact with a protein kinase C activator (*e.g*., bryostatin) in conjunction with a calcium ionophore. For co-stimulation of an accessory molecule on the surface of the T-cells, a ligand that binds the accessory molecule is used. For example, a population of CD4⁺ cells can be contacted with an anti-CD3 antibody and an anti-CD28 antibody, under conditions appropriate for stimulating proliferation of the T-cells. Similarly, to stimulate proliferation of CD8⁺ T-cells, an anti-CD3 antibody and the anti-CD28 antibody B-T3, XR-CD28 (Diaclone, Besançon, France) can be used as can other methods commonly known in the art (Berg et al., Transplant Proc. 30(8):3975-3977, 1998; Haanen et al., J. Exp. Med. 190(9):1319-1328, 1999; Garland et al., J. Immunol Meth. 227(1-2):53-63,1999).

The primary stimulatory signal and the co-stimulatory signal for the T-cell may be provided by different protocols. When coupled to a surface, the agents may be coupled to the same surface (*i.e*., in "cis" formation) or to separate surfaces (*i.e*., in "trans" formation). The agents may be in soluble form, and then cross-linked to a surface, such as a cell expressing FC receptors or an antibody or other binding agent which will bind to the agents. In a preferred embodiment, the two agents are immobilized on a spherical or semi-spherical surface, the prototypic examples being beads or cells, either on the same bead, *i.e*., "cis," or to separate beads, *i.e*., "trans." By way of example, the agent providing the primary activation signal is an anti-CD3 antibody and the agent providing the co-stimulatory signal is an anti-CD28 antibody; and both agents are co-immobilized to the same bead in equivalent molecular amounts. In one embodiment, a 1:1 ratio of each antibody bound to the beads for T-cell expansion and T-cell growth is used. In certain aspects of the present invention, a ratio of anti CD3:CD28 antibodies bound to the beads is used such that an increase in T cell expansion is observed as compared to the expansion observed using a ratio of 1:1. In one particular embodiment an increase of from about .5 to about 3 fold is observed as compared to the expansion observed using a ratio of 1:1. In one embodiment, the ratio of CD3:CD28 antibody bound to the beads ranges from 100:1 to 1:100 and all integer values there between. In one aspect of the present invention, more anti-CD28 antibody is bound to the particles than anti-CD3 antibody, *i.e*., the ratio of CD3:CD28 is less than one. In certain embodiments of the invention, the ratio of anti CD28 antibody to anti CD3 antibody bound to the beads is greater than 2:1. In one particular embodiment, a 1:100 CD3:CD28 ratio of antibody bound to beads is used. In another embodiment, a 1:75 CD3:CD28 ratio of antibody bound to beads is used. In a further embodiment, a 1:50 CD3:CD28 ratio of antibody bound to beads is used. In another embodiment, a 1:30 CD3:CD28 ratio of antibody bound to beads is used. In one preferred embodiment, a 1:10 CD3:CD28 ratio of antibody bound to beads is used. In another embodiment, a 1:3 CD3:CD28 ratio of antibody bound to the beads is used. In yet another embodiment, a 3:1 CD3:CD28 ratio of antibody bound to the beads is used.

Ratios of particles to cells from 1:500 to 500:1 and any integer values in between may be used to stimulate T-cells or other target cells. As those of ordinary skill in the art can readily appreciate, the ratio of particle to cells may depend on particle size relative to the target cell. For example, small sized beads could only bind a few cells, while larger beads could bind many. In certain embodiments the ratio of cells to particles ranges from 1:100 to 100:1 and any integer values in-between and in further embodiments the ratio comprises 1:9 to 9:1 and any integer values in between, can also be used to stimulate T-cells. The ratio of anti-CD3- and anti-CD28-coupled particles to T-cells that result in T-cell stimulation can vary as noted above, however certain preferred values include at least 1:5, 1:4, 1:3, 1:2, 1:1, 2:1, 3:1, 4:1 to 6:1, with one preferred ratio being at least 1:1 particles per T-cell. In one embodiment, a ratio of particles to cells of 1:1 or less is used. In further embodiments, the ratio of particles to cells can be varied depending on the day of stimulation. For example, in one embodiment, the ratio of particles to cells is from 1:1 to 10:1 on the first day and additional particles are added to the cells every day or every other day thereafter for up to 10 days, at final ratios of from 1:1 to 1:10 (based on cell counts on the day of addition). In one particular embodiment, the ratio of particles to cells is 1:1 on the first day of stimulation and adjusted to 1:5 on the third and fifth days of stimulation. In another embodiment, particles are added on a daily or every other day basis to a final ratio of 1:1 on the first day, and 1:5 on the third and fifth days of stimulation. In another embodiment, the ratio of particles to cells is 2:1 on the first day of stimulation and adjusted to 1:10 on the third and fifth days of stimulation. In another embodiment, particles are added on a daily or every other day basis to a final ratio of 1:1 on the first day, and 1:10 on the third and fifth days of stimulation. One of skill in the art will appreciate that a variety of other ratios may be suitable for use in the present invention. In particular, ratios will vary depending on particle size and on cell size and type.

Using certain methodologies it may be advantageous to maintain long-term stimulation of a population of T-cells following the initial activation and stimulation, by separating the T-cells from the stimulus after a period of about 12 to about 14 days. The rate of T-cell proliferation is monitored periodically (*e.g.,* daily) by, for example, examining the size or measuring the volume of the T-cells, such as with a Coulter Counter. In this regard, a resting T-cell has a mean diameter of about 6.8 microns, and upon initial activation and stimulation, in the presence of the stimulating ligand, the T-cell mean diameter will increase to over 12 microns by day 4 and begin to decrease by about day 6. When the mean T-cell diameter decreases to approximately 8 microns, the T-cells may be reactivated and re-stimulated to induce further proliferation of the T-cells. Alternatively, the rate of T-cell proliferation and time for T-cell restimulation can be monitored by assaying for the presence of cell surface molecules, such as, CD154, CD54, CD25, CD137, CD134, which are induced on activated T-cells.

For inducing long-term stimulation of a population of CD4⁺ and/or CD8⁺ T-cells, it may be necessary to reactivate and re-stimulate the T-cells with a stimulatory agent such as an anti-CD3 antibody and an anti-CD28 antibody (B-T3, XR-CD28 (Diaclone, Besançon, France)) or monoclonal antibody ES5.2D8 several times to produce a population of CD4⁺ or CD8⁺ cells increased in number from about 10 to about 1,000-fold the original T-cell population. For example, in one embodiment of the present invention, T-cells are stimulated as described in Example 1 for 2-3 times. In further embodiments, T-cells are stimulated as described in Example 1 for 4 or 5 times. Using the present methodology, it is possible to achieve T-cell numbers from about 100 to about 100,000-fold that have increased polyclonality as compared to prior to stimulation. Moreover, T-cells expanded by the method of the present invention secrete substantial levels of cytokines (*e.g*., IL-2, IFN-γ, IL-4, GM-CSF and TNF-α) into the culture supernatants. For example, as compared to stimulation with IL-2, CD4⁺ T-cells expanded by use of anti-CD3 and anti-CD28 co-stimulation secrete high levels of GM-CSF and TNF-α into the culture medium. These cytokines can be purified from the culture supernatants or the supernatants can be used directly for maintaining cells in culture. Similarly, the T-cells expanded by the method of the present invention together with the culture supernatant and cytokines can be administered to support the growth of cells *in vivo.*

In one embodiment, T-cell stimulation is performed, for example with anti-CD3 and anti-CD28 antibodies co-immobilized on beads (3x28 beads), for a period of time sufficient for the cells to return to a quiescent state (low or no proliferation) (approximately 8-14 days after initial stimulation). The stimulation signal is then removed from the cells and the cells are washed and infused back into the patient. The cells at the end of the stimulation phase are rendered "super-inducible" by the methods of the present invention, as demonstrated by their ability to respond to antigens and the ability of these cells to demonstrate a memory-like phenotype, as is evidence by the examples. Accordingly, upon re-stimulation either exogenously or by an antigen *in vivo* after infusion, the activated T-cells demonstrate a robust response characterized by unique phenotypic properties, such as sustained CD154 expression, increased cytokine production, etc.

In further embodiments of the present invention, the cells, such as T-cells are combined with agent-coated or conjugated beads, the beads and the cells are subsequently separated, and then the cells are cultured. In an alternative embodiment, prior to culture, the agent-coated or conjugated beads and cells are not separated but are cultured together. In a further embodiment, the beads and cells are first concentrated by application of a force, resulting in cell surface moiety ligation, thereby inducing cell stimulation and/or polarization of the activation signal.

By way of example, when T-cells are the target cell population, the cell surface moieties may be ligated by allowing paramagnetic beads to which anti-CD3 and anti-CD28 are attached (3x28 beads) to contact the T-cells prepared. In one embodiment the cells (for example, 10⁴ to 10⁹ T-cells) and beads (for example, DYNABEADS® M-450 CD3/CD28 T paramagnetic beads at a ratio of 1:1) are combined in a buffer, preferably PBS (without divalent cations such as, calcium and magnesium). Again, those of ordinary skill in the art can readily appreciate any cell concentration may be used. For example, the target cell may be very rare in the sample and comprise only 0.01 % of the sample or the entire sample (*i.e*., 100%) may comprise the target cell of interest Accordingly, any cell number is within the context of the present invention. In certain embodiments, it may be desirable to significantly decrease the volume in which particles and cells are mixed together (*i.e*., increase the concentration of cells), to ensure maximum contact of cells and particles. For example, in one embodiment, a concentration of about 2 billion cells/ml is used. In another embodiment, greater than 100 million cells/ml is used. In a further embodiment, a concentration of cells of 10, 15, 20, 25, 30, 35, 40, 45, or 50 million cells/ml is used. In yet another embodiment, a concentration of cells from 75, 80, 85, 90, 95, or 100 million cells/ml is used. In further embodiments, concentrations of 125 or 150 million cells/ml can be used. Using high concentrations can result in increased cell yield, cell activation, and cell expansion Further, use of high cell concentrations allows more efficient capture of cells that may weakly express target antigens of interest, such as CD28-negative T cells. Such populations of cells may have therapeutic value and would be desirable to obtain. For example, using high concentration of cells allows more efficient selection of CD8+ T cells that normally have weaker CD28 expression.

In a related embodiment, it may be desirable to use lower concentrations of cells. By significantly diluting the mixture of T cells and particles, interactions between particles and cells is minimized. This selects for cells that express high amounts of desired antigens to be bound to the particles. For example, CD4+ T cells express higher levels of CD28 and are more efficiently captured and stimulated than CD8+ T cells in dilute concentrations. In one embodiment, the concentration of cells used is about 5 X 10⁶/ml. In other embodiments, the concentration used can be from about 1 X 10⁵/ml to about 1 X 10⁶/ml, and any integer value in between.

The buffer that the cells are suspended in may be any that is appropriate for the particular cell type. When utilizing certain cell types the buffer may contain other components, *e.g*., 1-5% serum, necessary to maintain cell integrity during the process. In another embodiment, the cells and beads may be combined in cell culture media. The cells and beads may be mixed, for example, by rotation, agitation or any means for mixing, for a period of time ranging from one minute to several hours. The container of beads and cells is then concentrated by a force, such as placing in a magnetic field. Media and unbound cells are removed and the cells attached to the beads or other surface are washed, for example, by pumping via a peristaltic pump, and then resuspended in media appropriate for cell culture.

In one embodiment of the present invention, the mixture may be cultured for 30 minutes to several hours (about 3 hours) to about 14 days or any hourly or minute integer value in between. In another embodiment, the mixture may be cultured for 21 days. In one embodiment of the invention the beads and the T-cells are cultured together for about eight days. In another embodiment, the beads and T-cells are cultured together for 2-3 days. As described above, several cycles of stimulation may also be desired such that culture time of T cells can be 60 days or more. Conditions appropriate for T-cell culture include an appropriate media (*e.g*., Minimal Essential Media or RPMI Media 1640 or, X-vivo 15, (BioWhittaker)) that may contain factors necessary for proliferation and viability, including serum (*e.g*., fetal bovine or human serum) or interleukin-2 (IL-2). insulin, or any other additives for the growth of cells known to the skilled artisan. Media can include RPMI 1640, AIM-V, DMEM, MEM, α -MEM, F-12, X-Vivo 15, and X-Vivo 20, with added amino acids and vitamins, either serum-free or supplemented with an appropriate amount of serum (or plasma) or a defined set of hormones, and/or an amount of cytokine(s) sufficient for the growth and expansion of T-cells. Antibiotics, *e.g*., penicillin and streptomycin, are included only in experimental cultures, not in cultures of cells that are to be infused into a subject. The target cells are maintained under conditions necessary to support growth, for example, an appropriate temperature (*e.g*., 37° C) and atmosphere (*e.g*., air plus 5% C0₂).

In another embodiment, the time of exposure to stimulatory agents anti-CD3/anti-CD28 (*i.e*., 3x28)-coated beads may be modified or tailored in such a way to obtain a desired T-cell phenotype. Alternatively, a desired population of T-cells can be selected using any number of selection techniques, prior to stimulation. One may desire a greater population of helper T-cells (T_{H}), typically CD4⁺ as opposed to CD8⁺ cytotoxic or regulatory T-cells, because an expansion of T_{H} cells could improve or restore overall immune responsiveness. While many specific immune responses are mediated by CD8⁺ antigen-specific T-cells, which can directly lyse or kill target cells, most immune responses require the help of CD4⁺ T-cells, which express important immune-regulatory molecules, such as GM-CSF, CD40L, and IL-2, for example. Where CD4-mediated help if preferred, a method, such as that described herein, which preserves or enhances the CD4:CD8 ratio could be of significant benefit. Increased numbers of CD4⁺ T-cells can increase the amount of cell-expressed CD40L introduced into patients, potentially improving target cell visibility (improved APC function). Similar effects can be seen by increasing the number of infused cells expressing GM-CSF, or IL-2, all of which are expressed predominantly by CD4⁺ T-cells. Alternatively, in situations where CD4-help is needed less and increased numbers of CD8⁺ T-cells are desirous, the XCELLERATE^{™} approaches described herein (see Example 1) can also be utilized, by for example, pre-selecting for CD8⁺ cells prior to stimulation and/or culture. Such situations may exist where increased levels of IFN-γ or increased cytolysis of a target cell is preferred. One may also modify time and type of exposure to stimulatory agents to expand T cells with a desired TCR repertoire, *e.g*., expressing desired Vβ family genes.

To effectuate isolation of different T-cell populations, exposure times to the to the particles may be varied. For example, in one preferred embodiment, T-cells are isolated by incubation with 3x28 beads, such as Dynabeads M-450, for a time period sufficient for positive selection of the desired T cells. In one embodiment, the time period is about 30 minutes. In a further embodiment, the time period is at least 1, 2, 3, 4, 5, or 6 hours. In yet another preferred embodiment, the time period is 10 to 24 hours or more. In one preferred embodiment, the incubation time period is 24 hours. For isolation of T cells from cancer patients, use of longer incubation times, such as 24 hours, can increase cell yield.

In certain embodiments, stimulation and/or expansion times may be 10 weeks or less, 8 weeks or less, four weeks or less, 2 weeks or less, 10 days or less, or 8 days or less (four weeks or less includes all time ranges from 4 weeks down to 1 day (24 hours) or any value between these numbers). In some embodiments in may be desirable to clone T cells using, for example, limiting dilution or cell sorting, wherein longer stimulation time may be necessary. In some embodiments, stimulation and expansion may be carried out for 6 days or less, 4 days or less, 2 days or less, and in other embodiments for as little as 24 or less hours, and preferably 4-6 hours or less (these ranges include any integer values in between). When stimulation of T-cells is carried out for shorter periods of time, the population of T-cells may not increase in number as dramatically, but the population will provide more robust and healthy activated T-cells that can continue to proliferate *in vivo* and more closely resemble the natural effector T-cell pool. As the availability of T-cell help is often the limiting factor in antibody responses to protein antigens, the ability to selectively expand or selectively infuse a CD4⁺ rich population of T-cells into a subject is extremely beneficial. Further benefits of such enriched populations are readily apparent in that activated helper T-cells that recognize antigens presented by B lymphocytes deliver two types of stimuli, physical contact and cytokine production, that result in the proliferation and differentiation of B cells.

In the various embodiments, one of ordinary skill in the art understands removal of the stimulation signal from the cells is dependent upon the type of surface used. For example, if paramagnetic beads are used, then magnetic separation is the feasible option. Separation techniques are described in detail by paramagnetic bead manufacturers' instructions (for example, DYNAL Inc., Oslo, Norway). Furthermore, filtration may be used if the surface is a bead large enough to be separated from the cells. In addition, a variety of transfusion filters are commercially available, including 20 micron and 80 micron transfusion filters (Baxter). Accordingly, so long as the beads are larger than the mesh size of the filter, such filtration is highly efficient In a related embodiment, the beads may pass through the filter, but cells may remain, thus allowing separation. In one particular embodiment, the biocompatible surface used degrades (*i.e.,* is biodegradable) in culture during the exposure period.

Although the antibodies used in the methods described herein can be readily obtained from public sources, such as the ATCC, antibodies to T-cell accessory molecules and the CD3 complex can be produced by standard techniques. Methodologies for generating antibodies for use in the methods of the invention are well-known in the art and are discussed in further detail herein.

### LIGAND IMMOBILIZATION ON A SURFACE

As indicated above, the methods of the present invention preferably use ligands bound to a surface. The surface may be any surface capable of having a ligand bound thereto or integrated into and that is biocompatible, that is, substantially nontoxic to the target cells to be stimulated. The biocompatible surface may be biodegradable or non-biodegradable. The surface may be natural or synthetic, and a synthetic surface may be a polymer. The surface may comprise collagen, purified proteins, purified peptides, polysaccharides, glycosaminoglycans, extracellular matrix compositions, liposomes, or cells. A polysaccharide may include for example, cellulose, agarose, dextran, chitosan, hyaluronic acid, or alginate. Other polymers may include polyesters, polyethers, polyanhydrides, polyalkylcyanoacryllates, polyacrylamides, polyorthoesters, polyphosphazenes, polyvinylacetates, block copolymers, polypropylene, polytetrafluorethylene (PTFE), or polyurethanes. The polymer may be lactic acid or a copolymer. A copolymer may comprise lactic acid and glycolic acid (PLGA). Non-biodegradable surfaces may include polymers, such as poly(dimethylsiloxane) and poly(ethylene-vinyl acetate). Biocompatible surfaces include for example, glass (*e.g*., bioglass), collagen, chitin, metal, hydroxyapatite, aluminate, bioceramic materials, hyaluronic acid polymers, alginate, acrylic ester polymers, lactic acid polymer, glycolic acid polymer, lactic acid/glycolic acid polymer, purified proteins, purified peptides, or extracellular matrix compositions. Other polymers comprising a surface may include glass, silica, silicon, hydroxyapatite, hydrogels, collagen, acrolein, polyacrylamide, polypropylene, polystyrene, nylon, or any number of plastics or synthetic organic polymers, or the like. The surface may comprise a biological structure, such as a liposome or cell surface. The surface may be in the form of a lipid, a plate, bag, pellet, fiber, mesh, or particle. A particle may include, a colloidal particle, a microsphere, nanoparticle, a bead, or the like. In the various embodiments, commercially available surfaces, such as beads or other particles, are useful (*e.g*., Miltenyi Particles, Miltenyi Biotec, Germany; Sepharose beads, Pharmacia Fine Chemicals, Sweden; DYNABEADS™, Dynal Inc., New York; PURABEADS™, Prometic Biosciences).

When beads are used, the bead may be of any size that effectuates target cell stimulation In one embodiment, beads are preferably from about 5 nanometers to about 500 µm in size. Accordingly, the choice of bead size depends on the particular use the bead will serve. For example, if the bead is used for monocyte depletion, a small size is chosen to facilitate monocyte ingestion (*e.g*., 2.8 µm and 4.5 µm in diameter or any size that may be engulfed, such nanometer sizes); however, when separation of beads by filtration is desired, bead sizes of no less than 50 µm are typically used. Further, when using paramagnetic beads, the beads typically range in size from about 2.8 µm to about 500 µm and more preferably from about 2.8 µm to about 50 µm. Lastly, one may choose to use super-paramagnetic nanoparticles which can be as small as about 10 µm. Accordingly, as is readily apparent from the discussion above, virtually any particle size may be utilized.

An agent may be attached or coupled to, or integrated into a surface by a variety of methods known and available in the art. The agent may be a natural ligand, a protein ligand, or a synthetic ligand. The attachment may be covalent or noncovalent, electrostatic, or hydrophobic and may be accomplished by a variety of attachment means, including for example, chemical, mechanical, enzymatic, electrostatic, or other means whereby a ligand is capable of stimulating the cells. The attachment of the agent may be direct or indirect (*e.g*., tethered). For example, the antibody to a ligand first may be attached to a surface (direct attachment), or avidin or streptavidin, or a second antibody that binds the first, may be attached to the surface for binding to a biotinylated ligand (indirect attachment). The antibody to the ligand may be attached to the surface via an anti-idiotype antibody. Another example includes using protein A or protein G, or other non-specific antibody binding molecules, attached to surfaces to bind an antibody. Alternatively, the ligand may be attached to the surface by chemical means, such as cross-linking to the surface, using commercially available cross-linking reagents (Pierce, Rockford, IL) or other means. In certain embodiments, the ligands are covalently bound to the surface. Further, in one embodiment, commercially available tosyl-activated DYNABEADS™ or DYNABEADS™ with epoxy-surface reactive groups are incubated with the polypeptide ligand of interest according to the manufacturer's instructions. Briefly, such conditions typically involve incubation in a phosphate buffer from pH 4 to pH 9.5 at temperatures ranging from 4 to 37 degrees C.

In one aspect, the agent, such as certain ligands may be of singular origin or multiple origins and may be antibodies or fragments thereof while in another aspect, when utilizing T-cells, the co-stimulatory ligand is a B7 molecule (*e.g*., B7-1, B7-2). These ligands are coupled to the surface by any of the different attachment means discussed above. The B7 molecule to be coupled to the surface may be isolated from a cell expressing the co-stimulatory molecule, or obtained using standard recombinant DNA technology and expression systems that allow for production and isolation of the co-stimulatory molecule(s) as described herein. Fragments, mutants, or variants of a B7 molecule that retain the capability to trigger a co-stimulatory signal in T-cells when coupled to the surface of a cell can also be used. Furthermore, one of ordinary skill in the art will recognize that any ligand useful in the activation and induction of proliferation of a subset of T-cells may also be immobilized on beads or culture vessel surfaces or any surface. In addition, while covalent binding of the ligand to the surface is one preferred methodology, adsorption or capture by a secondary monoclonal antibody may also be used. The amount of a particular ligand attached to a surface may be readily determined by flow cytometric analysis if the surface is that of beads or determined by enzyme-linked immunosorbant assay (ELISA) if the surface is a tissue culture dish, mesh, fibers, bags, for example.

In a particular embodiment, the stimulatory form of a anti-CD28 antibody or fragment thereof is attached to the same solid phase surface as the agent that stimulates the TCR/CD3 complex, an anti-CD3 antibody. In addition to anti-CD3 antibodies, other antibodies that bind to receptors that mimic antigen signals may be used. For example, the beads or other surfaces may be coated with combinations of anti-CD2 antibodies and a B7 molecule and in particular anti-CD3 antibodies and anti-CD28 antibodies.

When coupled to a surface, the agents may be coupled to the same surface (*i.e*., in "cis" formation) or to separate surfaces (*i.e*., in "trans" formation). Alternatively, one agent may be coupled to a surface and the other agent in solution. In one embodiment, the agent providing the co-stimulatory signal is bound to a cell surface and the agent providing the primary activation signal is in solution or coupled to a surface. In a preferred embodiment, the two agents are immobilized on beads, either on the same bead, *i.e*., "cis," or to separate beads, *i.e*., "trans." By way of example, the agent providing the primary activation signal is an anti-CD3 antibody and the agent providing the co-stimulatory signal is an anti-CD28 antibody; and both agents are co-immobilized to the same bead in equivalent molecular amounts. In one embodiment, a 1:1 ratio of each antibody bound to the beads for CD4⁺ T-cell expansion and T-cell growth is used. In certain aspects of the present invention, a ratio of anti CD3:CD28 antibodies bound to the beads is used such that an increase in T cell expansion is observed as compared to the expansion observed using a ratio of 1:1. In one particular embodiment an increase of from about .5 to about 3 fold is observed as compared to the expansion observed using a ratio of 1:1. In one embodiment, the ratio of CD3:CD28 antibody bound to the beads ranges from 100:1 to 1:100 and all integer values there between. In one aspect of the present invention, more anti-CD28 antibody is bound to the particles than anti-CD3 antibody, *i.e*., the ratio of CD3:CD28 is less than one. In certain embodiments of the invention, the ratio of anti CD28 antibody to anti CD3 antibody bound to the beads is greater than 2:1. In one particular embodiment, a 1:100 CD3:CD28 ratio of antibody bound to beads is used. In another embodiment, a 1:75 CD3:CD28 ratio of antibody bound to beads is used. In a further embodiment, a 1:50 CD3:CD28 ratio of antibody bound to beads is used. In another embodiment, a 1:30 CD3:CD28 ratio of antibody bound to beads is used. In one preferred embodiment, a 1:10 CD3:CD28 ratio of antibody bound to beads is used. In another embodiment, a 1:3 CD3:CD28 ratio of antibody bound to the beads is used. In yet another embodiment, a 3:1 CD3:CD28 ratio of antibody bound to the beads is used.

### AGENTS

The antibodies and fragments thereof may be produced in accordance with conventional techniques, such as hybridoma methods and recombinant DNA and protein expression techniques. Useful antibodies and fragments may be derived from any species, including humans, or may be formed as chimeric proteins, which employ sequences from more than one species.

Methods well known in the art may be used to generate antibodies, polyclonal antisera, or monoclonal antibodies that are specific for a ligand. Antibodies also may be produced as genetically engineered immunoglobulins (Ig) or Ig fragments designed to have desirable properties. For example, by way of illustration and not limitation, antibodies may include a recombinant IgG that is a chimeric fusion protein having at least one variable (V) region domain from a first mammalian species and at least one constant region domain from a second distinct mammalian species. Most commonly, a chimeric antibody has murine variable region sequences and human constant region sequences. Such a murine/human chimeric immunoglobulin may be "humanized" by grafting the complementarity determining regions (CDRs), which confer binding specificity for an antigen, derived from a murine antibody into human-derived V region framework regions and human-derived constant regions. Antibodies containing CDRs of different specificities can also be combined to generate multi-specific (bi or tri-specific, etc.) antibodies. Fragments of these molecules may be generated by proteolytic digestion, or optionally, by proteolytic digestion followed by mild reduction of disulfide bonds and alkylation, or by recombinant genetic engineering techniques.

Antibodies are defined to be "immunospecific" if they specifically bind the antigen with an affinity constant, Kₐ, of greater than or equal to about 10⁴ M⁻¹, preferably of greater than or equal to about 10⁵ M⁻¹, more preferably of greater than or equal to about 10⁶ M⁻¹, and still more preferably of greater than or equal to about 10⁷ M⁻¹. Affinities of binding partners or antibodies can be readily determined using conventional techniques, for example, those described by Scatchard et al. (Ann N.Y. Acad Sci. USA 51:660, 1949) or by surface plasmon resonance (BIAcore, Biosensor, Piscataway, NJ) *See, e.g.,* Wolff et al., Cancer Res., 53:2560-2565, 1993).

Antibodies may generally be prepared by any of a variety of techniques known to those having ordinary skill in the art *(See, e.g.,* Harlow et al., Antibodies: A Laboratory Manual, 1988, Cold Spring Harbor Laboratory). In one such technique, an animal is immunized with the ligand as antigen to generate polyclonal antisera. Suitable animals include rabbits, sheep, goats, pigs, cattle, and may include smaller mammalian species, such as, mice, rats, and hamsters. Antibodies of the present invention may also be generated as described in U.S. patent Nos: 6,150,584, 6,130,364, 6,114,598, 5,833,985, 6,071,517, 5,756,096, 5,736,137, and 5,837,243.

An immunogen may be comprised of cells expressing the ligand, purified or partially purified ligand polypeptides or variants or fragments thereof, or ligand peptides. Ligand peptides may be generated by proteolytic cleavage or may be chemically synthesized. Peptides for immunization may be selected by analyzing the primary, secondary, or tertiary structure of the ligand according to methods know to those skilled in the art in order to determine amino acid sequences more likely to generate an antigenic response in a host animal (*See, e.g.,* Novotny, Mol. Immunol. 28:201-207, 1991; Berzoksky, Science 229:932-40, 1985).

Preparation of the immunogen may include covalent coupling of the ligand polypeptide or variant or fragment thereof, or peptide to another immunogenic protein, such as, keyhole limpet hemocyanin or bovine serum albumin. In addition, the peptide, polypeptide, or cells may be emulsified in an adjuvant (*See* Harlow et al., Antibodies: A Laboratory Manual, 1988 Cold Spring Harbor Laboratory). In general, after the first injection, animals receive one or more booster immunizations according to a preferable schedule for the animal species. The immune response may be monitored by periodically bleeding the animal, separating the sera, and analyzing the sera in an immunoassay, such as an Ouchterlony assay, to assess the specific antibody titer. Once an antibody titer is established, the animals may be bled periodically to accumulate the polyclonal antisera. Polyclonal antibodies that bind specifically to the ligand polypeptide or peptide may then be purified from such antisera, for example, by affinity chromatography using protein A or using the ligand polypeptide or peptide coupled to a suitable solid support.

Monoclonal antibodies that specifically bind ligand polypeptides or fragments or variants thereof may be prepared, for example, using the technique of Kohler and Milstein (nature, 256:495-497, 1975; Eur. J. Immunol. 6:511-519, 1976) and improvements thereto. Hybridomas, which are immortal eucaryotic cell lines, may be generated that produce antibodies having the desired specificity to a ligand polypeptide or variant or fragment thereof. An annmal-for example, a rat, hamster, or preferably mouse-is immunized with the ligand immunogen prepared as described above. Lymphoid cells, most commonly, spleen cells, obtained from an immunized animal may be immortalized by fusion with a drug-sensitized myeloma cell fusion partner, preferably one that is syngeneic with the immunized animal. The spleen cells and myeloma cells may be combined for a few minutes with a membrane fusion-promoting agent, such as polyethylene glycol or a nonionic detergent, and then plated at low density on a selective medium that supports the growth of hybridoma cells, but not myeloma cells. A preferred selection media is HAT (hypoxanthine, aminopterin, thymidine). After a sufficient time, usually about 1 to 2 weeks, colonies of cells are observed. Single colonies are isolated, and antibodies produced by the cells may be tested for binding activity to the ligand polypeptide or variant or fragment thereof. Hybridomas producing antibody with high affinity and specificity for the ligand antigen are preferred Hybridomas that produce monoclonal antibodies that specifically bind to a ligand polypeptide or variant or fragment thereof are contemplated by the present invention.

Monoclonal antibodies may be isolated from the supernatants of hybridoma cultures. An alternative method for production of a murine monoclonal antibody is to inject the hybridoma cells into the peritoneal cavity of a syngeneic mouse. The mouse produces ascites fluid containing the monoclonal antibody. Contaminants may be removed from the antibody by conventional techniques, such as chromatography, gel filtration, precipitation, or extraction.

Human monoclonal antibodies may be generated by any number of techniques. Methods include but are not limited to, Epstein Barr Virus (EBV) transformation of human peripheral blood cells (*see,* U. S. Patent No. 4,464,456), *in vitro* immunization of human B cells (*see, e.g.,* Boerner et al., J. Immunol. 147:86-95, 1991), fusion of spleen cells from immunized transgenic mice carrying human immunoglobulin genes and fusion of spleen cells from immunized transgenic mice carrying immunoglobulin genes inserted by yeast artificial chromosome (YAC) (*see, e.g*., U. S. Patent No. 5,877,397; Bruggemann et al., Curr. Opin. Biotechnol. 8:455-58, 1997; Jakobovits et al., Ann. N. Y. Acad Sci. 764:525-35, 1995), or isolation from human immunoglobulin V region phage libraries.

Chimeric antibodies and humanized antibodies for use in the present invention may be generated. A chimeric antibody has at least one constant region domain derived from a first mammalian species and at least one variable region domain derived from a second distinct mammalian species (*See, e.g.,* Morrison et al., Proc. Natl. Acad. Sci. USA, 81:6851-55, 1984). Most commonly, a chimeric antibody may be constructed by cloning the polynucleotide sequences that encode at least one variable region domain derived from a non-human monoclonal antibody, such as the variable region derived from a murine, rat, or hamster monoclonal antibody, into a vector containing sequences that encode at least one human constant region. (*See, e.g.,* Shin et al., Methods Enzymol. 178:459-76, 1989; Walls et al., Nucleic Acids Res. 21:2921-29, 1993). The human constant region chosen may depend upon the effector functions desired for the particular antibody. Another method known in the art for generating chimeric antibodies is homologous recombination (U.S. Patent No. 5,482,856). Preferably, the vectors will be transfected into eukaryotic cells for stable expression of the chimeric antibody.

A non-human/human chimeric antibody may be further genetically engineered to create a "humanized" antibody. Such an antibody has a plurality of CDRs derived from an immunoglobulin of a non-human mammalian species, at least one human variable framework region, and at least one human immunoglobulin constant region. Humanization may yield an antibody that has decreased binding affinity when compared with the non-human monoclonal antibody or the chimeric antibody. Those having skill in the art, therefore, use one or more strategies to design humanized antibodies.

Within certain embodiments, the use of antigen-binding fragments of antibodies may be preferred. Such fragments include Fab fragments or F(ab')₂ fragments, which may be prepared by proteolytic digestion with papain or pepsin, respectively. The antigen binding fragments may be separated from the Fc fragments by affinity chromatography, for example, using immobilized protein A or immobilized ligand polypeptide or a variant or a fragment thereof. An alternative method to generate Fab fragments includes mild reduction of F(ab')₂ fragments followed by alkylafion *(See, e.g.,* Weir, Handbook of Experimental Immunology, 1986, Blackwell Scientific, Boston).

Non-human, human, or humanized heavy chain and light chain variable regions of any of the above described Ig molecules may be constructed as single chain Fv (sFv) fragments (single chain antibodies). *See, e.g.*, Bird et al., Science 242:423-426, 1988; Huston et al., Proc. Natl. Acad Sci. USA 85:5879-5883, 1988. Multi-functional fusion proteins may be generated by linking polynucleotide sequences encoding an sFv in-frame with polynucleotide sequences encoding various effector proteins. These methods are known in the art, and are disclosed, for example, in EP-B1-0318554, U.S. Patent No. 5,132,405, U.S. Patent No. 5,091,513, and U.S. Patent No. 5,476,786.

An additional method for selecting antibodies that specifically bind to a ligand polypeptide or variant or fragment thereof is by phage display (*See, e.g.,* Winter et al., Annul. Rev. Immunol. 12:433-55, 1994; Burton et al., Adv. Immunol. 57:191-280, 1994). Human or marine immunoglobulin variable region gene combinatorial libraries may be created in phage vectors that can be screened to select Ig fragments (Fab, Fv, sFv, or multimers thereof) that bind specifically to a ligand polypeptide or variant or fragment thereof (*See*, *e*.*g*., U.S. Patent No. 5,223,409; Huse et al., Science 246:1275-81, 1989; Kang et al., Proc. Natl. Acad. Sci. USA 88:4363-66, 1991; Hoogenboom et al., J. Molec. Biol. 227:381-388, 1992; Schlebusch et al., Hybridoma 16:47-52, 1997 and references cited therein).

### METHODS OF USE

In addition to the methods described above, cells stimulated and/or activated by the methods herein described may be utilized in a variety of contexts. The T cells with increased polyclonality of the present invention can be infused into any individual with a condition where a skewed T cell repertoire is suspected or observed. The T cells with increased polyclonality of the present invention can be infused into donors to provide broad and potent immune protection. The compositions and methods described herein can be used to treat an immunocompromised individual, e.g., an individual with an immunological defect, or a skewed T cell repertoire as described herein (either naturally occurring or artificially induced by a drug or therapy).

In certain embodiments, the immunocompromised individual is immunocompromised naturally, *i*.*e*., due to naturally occurring causes, such as by any of the diseases or disorders described herein. In other embodiments, an individual reaches an immunocompromised state by induction, for example as a result of any number of treatments for any of the diseases described herein. Within this context, an individual can be immunocompromised, or in other words, may have an immunological defect, or a skewed T cell repertoire, as a result of chemotherapy, treatments typically administered in the context of transplantation, cytotoxic agents, immunosuppressive agents or any other treatments that lead to an altered or skewed T cell repertoire as described herein. In certain embodiments, individuals are immunocompromised as a result of chemotherapy, radiation, or treatment with agents such as cyclosporin, azathioprine, methotrexate, mycophenolate, and FK506, antibodies, or other immunoablative agents such as CAMPATH, anti-CD3 antibodies, cytoxin, fludaribine, cyclosporin, FK506, rapamycin, mycophenolic acid, steroids, FR901228, and irradiation. These drugs inhibit either the calcium dependent phosphatase calcineurin (cyclosporine and FK506) or inhibit the p70S6 kinase that is important for growth factor induced signaling (rapamycin). (Liu et al., Cell 66:807-815, 1991; Henderson et al., Immun. 73:316-321, 1991; Bierer et al., Curr. Opin. Immun. 5:763-773, 1993; Isoniemi (supra)).

Naturally occurring immune responses single out a few immunodominant epitopes for any given antigen and the T cells with specificity for these epitopes are activated, expand, and mediate immune responses. Unfortunately, many other potential epitopes fail to compete in the *in vivo* T cell activation process and remain non-activated/non-participatory in the immune response, thereby increasing the likelihood that immune surveillance can be overpowered by the pathogen/tumor. By activating and increasing the polyclonality of a donor's T cells, these less dominant T cells with TCR's capable of responding to target antigens, can be driven to a state of improved responsiveness making them potential players in an immune response. This broadens the immune system's armamentarium of T cells with different specificities to challenge any immunological insults. This approach thus serves to help protect against escape variants that occur when narrow immune responses are the mode of action.

The methodologies described herein can be used to selectively expand a population of CD28⁺, CD4⁺, CD8⁺, CD45RA⁺, and/or CD45RO⁺ T-cells with increased polyclonality in terms of TCR expression for use in the treatment of infectious diseases, autoimmune diseases, any number of cancers, hematological disease (*e.g*., cytopenias), concurrent with transplantation (*e.g*., hematopoietic stem cell transplantation) or any of a variety of states or conditions of immunodeficiency, and for use in immunotherapy. As a result, a population of T-cells, which express TCRs that are polyclonal with respect to antigen reactivity, but essentially homogeneous with respect to either CD4⁺ or CD8⁺ can be produced. In addition, the method allows for the expansion of a population of T-cells in numbers sufficient to reconstitute an individual's total CD4⁺ or CD8⁺ T-cell population (the population of lymphocytes in an individual is approximately 5 X 10¹¹). The resulting T-cell population can also be genetically transduced and used for immunotherapy or can be used in methods of *in vitro* analyses of infectious agents. For example, a population of tumor-infiltrating lymphocytes can be obtained from an individual afflicted with cancer and the T-cells stimulated to proliferate to sufficient numbers. The resulting T-cell population can be genetically transduced to express tumor necrosis factor (TNF) or other proteins (for example, any number of cytokines, inhibitors of apoptosis (*e.g*., Bcl-2), genes that protect cells from HIV infection such as RevM10 or intrakines, and the like, targeting molecules, adhesion and/or homing molecules and any variety of antibodies or fragments thereof (*e.g*., Scfv)) and given to the individual.

One particular use for the CD4⁺ T-cells populations of the invention is the treatment of HIV infection in an individual. Prolonged infection with HIV eventually results in a marked decline in the number of CD4⁺ T lymphocytes. This decline, in turn, causes a profound state of immunodeficiency, rendering the patient susceptible to an array of life threatening opportunistic infections. Replenishing the number of CD4⁺ T-cells to normal levels may be expected to restore immune function to a significant degree. Thus, the method described herein provides a means for increasing the polyclonality of and expanding CD4⁺ T-cells to sufficient numbers to reconstitute this population in an HIV infected patient. It may also be necessary to avoid infecting the T-cells during long-term stimulation or it may desirable to render the T-cells permanently resistant to HIV infection. There are a number of techniques by which T-cells may be rendered either resistant to HIV infection or incapable of producing virus prior to restoring the T-cells to the infected individual. For example, one or more anti-retroviral agents can be cultured with CD4⁺ T-cells prior to expansion to inhibit HIV replication or viral production (*e.g*., drugs that target reverse transcriptase and/or other components of the viral machinery, see *e.g*., Chow et al. Nature 361:650-653, 1993).

Several methods can be used to genetically transduce T-cells to produce molecules which inhibit HIV infection or replication. For example, in various embodiments, T-cells can be genetically transduced to produce transdominant inhibitors, "molecular decoys", antisense molecules, or toxins. Such methodologies are described in further detail in U.S. Patent Application Nos. 08/253,751, 08/253,964, and PCT Publication No. WO 95/33823..

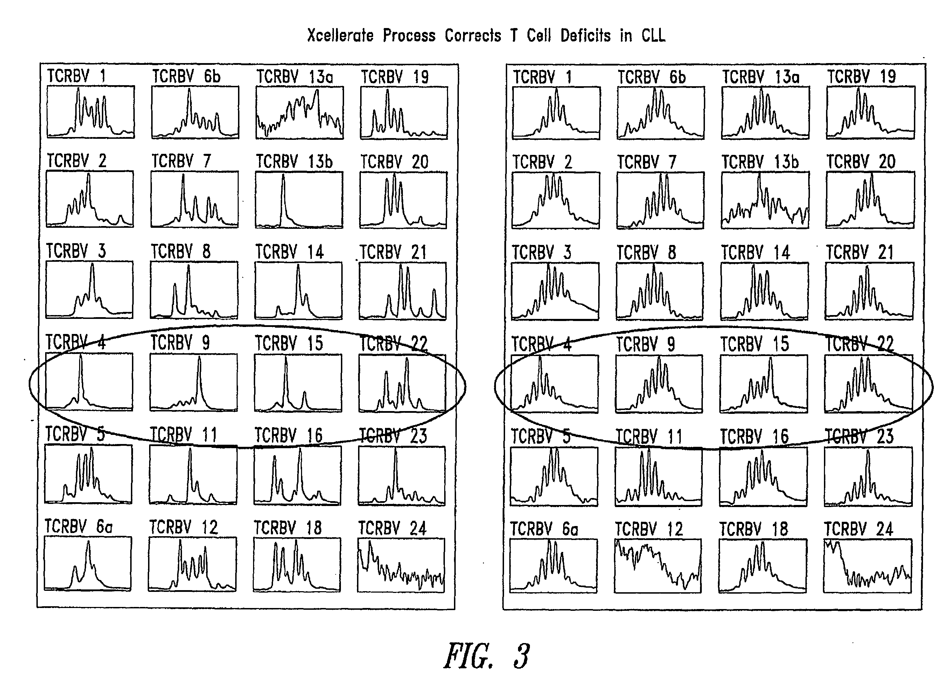

In one embodiment, malignancies such as non-Hodgkins lymphoma (NHL) and B-cell chronic, lymphocytic leukemia (B-CLL) can be treated. While initial studies using expanded T-cells have been tested in NHL (see Liebowitz et al., Curr. Opin. Onc. 10:533-541, 1998), the T-cell populations of the present invention offer increased polyclonal characteristics that can dramatically enhance the success of immunotherapy and reactivity. As shown in Figure 3, patients with B-CLL have a monoclonal or oligoclonal expression of TCRs within the T cell population for several Vβ families. Following a 12 day XCELLERATE^{™} process, polyclonality of TCR expression is restored to these T cell populations. Additionally, patients with B-CLL present special difficulties, including low relative T-cell numbers with high leukemic cell burden in the peripheral blood, accompanied by a general T-cell immunosuppression. The T-cell populations produced by the present invention can provide dramatically improved efficacy in treating this disease and especially when combined with stem cell (CD34⁺) transplantation therapy. Accordingly, increasing T-cell function and anti-CLL T-cell activity with anti-CD3 x anti-CD28 co-immobilized beads would be beneficial.

The cancers contemplated by the present disclosure, against which the immune response is induced, or which is to be prevented, inhibited, or reduced in presence, may include but are not limited to melanoma, non-Hodgkin's lymphoma, Hodgkin's disease, leukemia, plasmocytoma, sarcoma, glioma, thymoma, breast cancer, prostate cancer, colo-rectal cancer, kidney cancer, renal cell carcinoma, pancreatic cancer, nasopharyngeal carcinoma, esophageal cancer, brain cancer, lung cancer, ovarian cancer, cervical cancer, multiple myeloma, heptocellular carcinoma, acute lymphoblastic leukemia (ALL), acute myelogenous leukemia (AML), chronic myelogenous leukemia (CML), large granular lymphocyte leukemia (LGL), and chronic lymphocytic leukemia (CLL). In one embodiment, the cancer is B-cell chronic lymphocytic leukemia.

The methods of the present invention can also be used to restore immune responsiveness in individuals who have been treated with chemotherapy, cytotoxic agents, or any immunosuppressive agent as described herein and known to those of still in the art. In a further embodiment, the compositions and methods of the present invention can be used to treat (*i*.*e*., restore immune responsiveness in) individuals who have undergone hematopoeitic stem cell transplantation. In certain embodiments, individuals to be treated with the compositions of the present invention have received cord blood, allogeneic, autologous, or xenogeneic cell transplants.

In a further embodiment, the methods of the present invention can be used to restore immune responsiveness in individuals who have undergone gene therapy, or any procedure involving gene transduction which can lead to skewing of the T cell repertoire. More specifically, retroviral-mediated gene transfer in primary T lymphocytes can induce an activation and transduction/selection-dependent TCR Vβ skewing in gene modified cells. However, activation and stimulation of cells following gene modification using the methods described herein (*e*.*g*., CD3/CD28 costimulation as described herein), prevents the alterations (skewing) of TCR Vβ repertoire in both CD4 and CD8 T cell subsets.

In certain embodiments, the methods of the present invention can be used to restore or otherwise improve immune responsiveness in individuals afflicted with any number of disorders associated with immune dysfunction, including altered T cell repertoire, including but not limited to, diseases such as, rheumatoid arthritis, multiple sclerosis, insulin dependent diabetes, Addison's disease, celiac disease, chronic fatigue syndrome, inflammatory bowel disease, ulcerativecolitis, Crohn's disease, Fibromyalgia, systemic lupus erythematosus, psoriasis, Sjogren's syndrome, hyperthyroidism/Graves disease, hypothyroidism/Hashimoto's disease, Insulin-dependent diabetes (type 1), Myasthenia Gravis, endometriosis, scleroderma, pernicious anemia, Goodpasture syndrome, Wegener's disease, glomerulonephritis, aplastic anemia, any of a variety of cytopenias, paroxysmal nocturnal hemoglobinuria, myelodysplastic syndrome, idiopathic thrombocytopenic purpura, autoimmune hemolytic anemia, Fanconi anemia, Evan's syndrome, Factor VIII inhibitor syndrome, Factor IX inhibitor syndrome, systemic vasculitis, dermatomyositis, polymyositis and rheumatic fever. The methods and compositions described herein can be used to treat hematological disorders characterized by low blood counts.

In certain embodiments, the methods of the present invention can be used in the treatment of neurological disorders associated with T cell repertoire skewing.

T-cells can be stimulated and expanded as described herein to induce or enhance responsiveness to pathogenic agents, such as viruses (*e*.*g*., human immunodeficiency virus), bacteria, parasites and fungi. Pathogenic agents include any disease that is caused by an infectious organism. Infectious organisms may comprise viruses, (*e*.*g*., single stranded RNA viruses, single stranded DNA viruses, human immunodeficiency virus (HIV), hepatitis A, B, and C virus, herpes simplex virus (HSV), cytomegalovirus (CMV) Epstein-Barr virus (EBV), human papilloma virus (BPV)), parasites (*e*.*g*., protozoan and metazoan pathogens such as *Plasmodia* species, *Leishmania* species, *Schistosoma* species, *Trypanosoma* species), bacteria (*e*.*g*., *Mycobacteria*, in particular, *M*. *tuberculosis*, *Salmonella*, *Streptococci*, *E*. *coli*, *Staphylococci*), fungi (*e*.*g*.*, Candida* species, *Aspergillus* species), *Pneumocystis carinii*, and prions (known prions infect animals to cause scrapie, a transmissible, degenerative disease of the nervous system of sheep and goats, as well as bovine spongiform encephalopathy (BSE), or "mad cow disease", and feline spongiform encephalopathy of cats. Four prion diseases known to affect humans are kuru, Creutzfeldt-Jakob Disease (CJD), Gerstmann-Straussler-Scheinker Disease (GSS), and fatal familial insomnia (FFI)). As used herein "prion" includes all forms of prions causing all or any of these diseases or others in any animals used--and in particular in humans and domesticated farm animals.

T-cells can be stimulated and expanded as described herein to induce or enhance responsiveness in an immunocompromised individual, for example, an individual who has a congenital genetic disorder such as severe combined immunodeficiency (SCID) or common variable immunodeficiency (CVID). In one embodiment, T-cells can be stimulated and expanded as described herein to induce or enhance responsiveness in an individual who is immunocompromised as a result of treatment associated with bone marrow transplantation, chemotherapy, radiation therapy or other cancer treatment. In one embodiment, T-cells can be stimulated and expanded as described herein to induce or enhance responsiveness in an immunocompromised individual who has an immunodeficiency or an autoimmune disease. In yet a further embodiment, T-cells can be stimulated and expanded to induce or enhance responsiveness in an immunocompromised individual who has a chronic disease affecting the kidney, liver, or the pancreas. In one particular embodiment, the T cells of the present invention are used to induce or enhance responsiveness in an individual who has diabetes. In another embodiment, the T cells of the present invention are used to induce or enhance responsiveness in an individual who is affected by old age.

The present invention further provides a method for selectively expanding a population of T cells expressing a specific Vβ, Vα, Vγ, or Vδ gene. For example, in this method, T cells expressing a particular Vβ, Vα, Vγ, or Vδ gene are positively or negatively selected and then further expanded/stimulated according to the methods of the present invention. Alternatively, stimulated and expanded T cells expressing a particular Vβ, Vα, Vγ, or Vδ gene of interest can be positively or negatively selected and further stimulated and expanded.

In another example, blood is drawn into a stand-alone disposable device directly from the patient that contains two or more immobilized antibodies (*e*.*g*., anti-CD3 and anti-CD28) or other components to stimulate receptors required for T-cell activation prior to the cells being administered to the subject (*e*.*g*., immobilized on plastic surfaces or upon separable microparticles). In one embodiment, the disposable device may comprise a container (*e*.*g*., a plastic bag, or flask) with appropriate tubing connections suitable for combing/docking with syringes and sterile docking devices. This device will contain a solid surface for immobilization of T-cell activation components (*e*.*g*., anti-CD3 and anti-CD28 antibodies); these may be the surfaces of the container itself or an insert and will typically be a flat surface, an etched flat surface, an irregular surface, a porous pad, fiber, clinically acceptable/safe ferro-fluid, beads, etc.). Additionally when using the stand-alone device, the subject can remain connected to the device, or the device can be separable from the patient Further, the device may be utilized at room temperature or incubated at physiologic temperature using a portable incubator.

As devices and methods for collecting and processing blood and blood products are well known, one of skill in the art would readily recognize that given the teachings provided herein, that a variety of devices that fulfill the needs set forth above may be readily designed or existing devices modified. Accordingly, as such devices and methods are not limited by the specific embodiments set forth herein, but would include any device or methodology capable of maintaining sterility and which maintains blood in a fluid form in which complement activation is reduced and wherein components necessary for T-cell activation (*e*.*g*., anti-CD3 and anti-CD28 antibodies or ligands thereto) may be immobilized or separated from the blood or blood product prior to administration to the subject. Further, as those of ordinary skill in the art can readily appreciate a variety of blood products can be utilized in conjunction with the devices and methods described herein. For example, the methods and devices could be used to provide rapid activation of T-cells from cryopreserved whole blood, peripheral blood mononuclear cells, other cyropreserved blood-derived cells, or cryopreserved T-cell lines upon thaw and prior to subject administration, In another example, the methods and devices can be used to boost the activity of a previously ex vivo expanded T-cell product or T-cell line prior to administration to the subject, thus providing a highly activated T-cell product. Lastly, as will be readily appreciated the methods and devices above may be utilized for autologous or allogeneic cell therapy simultaneously with the subject and donor.

The methods of the present invention may also be utilized with vaccines to enhance reactivity of the antigen and enhance *in vivo* effect. Further, given that T-cells expanded by the present invention have a relatively long half-life in the body, these cells could act as perfect vehicles for gene therapy, by carrying a desired nucleic acid sequence of interest and potentially homing to sites of cancer, disease, or infection. Accordingly, the cells expanded by the present invention may be delivered to a patient in combination with a vaccine, one or more cytokines, one or more therapeutic antibodies, etc. Virtually any therapy that would benefit by a more robust T-cell population is within the context of the methods of use described herein.

The present invention provides methods for providing T cells with increased polyclonality in TCR expression for use in preventing, inhibiting, or reducing the presence of such cancers as, but not limited to, melanoma, non-Hodgkin's lymphoma, Hodgkin's disease, nasopharyngeal carcinoma, leukemia, plasmocytoma, sarcoma, glioma, thymoma, breast cancer, prostate cancer, colo-rectal cancer, kidney cancer, renal cell carcinoma, pancreatic cancer, esophageal cancer, brain cancer, lung cancer, ovarian cancer, cervical cancer, multiple myeloma, heptocellular carcinoma, acute lymphoblastic leukemia (ALL), acute myelogenous leukemia (AML), chronic myelogenous leukemia (CML), chronic lymphocytic leukemia (CLL), large granular lymphocyte leukemia (LGL), and other neoplasms known in the art.

Alternatively, the polyclonal T cell compositions as described herein can be used to induce or enhance responsiveness to an infectious organism. Infectious organisms may comprise a virus, such as a single stranded RNA virus or a single stranded DNA virus, human immunodeficiency virus (HIV), hepatitis A, B, or C virus, herpes simplex virus (HSV), human papilloma virus (HPV), cytomegalovirus (CMV), Epstein-Barr virus (EBV), a parasite, a bacterium, *M. tuberculosis, Pneumocystis carinii, Candida,* or *Aspergillus* or a combination thereof.

In another embodiment of the present invention, the polyclonal T cell compositions as described herein can be used to induce or enhance responsiveness to correct a congenital genetic disorder or an immunodeficiency disorder such as severe combined immunodeficiency (SCID) or common variable immunodeficiency (CVID). In certain embodiments, the polyclonal T cell compositions as described herein can be used to induce or enhance responsiveness to correct an immunodeficiency that is the result of treatment associated with bone marrow transplantation, chemotherapy, radiation therapy or other cancer treatment. In a further embodiment, the polyclonal T cell compositions as described herein can be used to induce or enhance responsiveness to correct an immunodeficiency or an autoimmune disease. In yet a further embodiment, the polyclonal T cell compositions as described herein can be used to induce or enhance responsiveness to correct a chronic disease affecting the kidney, liver, or the pancreas. In yet another embodiment, the polyclonal T cell compositions as described herein can be used to induce or enhance responsiveness to treat diabetes. In one certain embodiment, the polyclonal T cell compositions as described herein can be used to induce or enhance responsiveness to correct for immunodeficiencies associated with aging.

In a further embodiment, the T cell compositions showing increased polyclonality of TCR expression of the present invention can be used in conjunction with other therapies traditionally utilized for the treatment of such infectious diseases and cancers.

### PHARMACEUTICAL COMPOSITIONS

T-cell populations produced by the methods of the present invention may be administered either alone, or as a pharmaceutical composition in combination with diluents and/or with other components such as IL-2 or other cytokines or cell populations. Briefly, pharmaceutical compositions may comprise a target cell population as described herein, in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (*e*.*g*., aluminum hydroxide); and preservatives. Compositions are preferably formulated for intravenous administration.

Pharmaceutical compositions may be administered in a manner appropriate to the disease to be treated (or prevented). The quantity and frequency of administration will be determined by such factors as the condition of the patient, and the type and severity of the patient's disease, although appropriate dosages may be determined by clinical trials.

The immune response induced in the animal by administering the subject compositions of the present invention may include cellular immune responses mediated by cytotoxic T cells, capable of killing tumor and infected cells, and helper T cell responses. Humoral immune responses, mediated primarily by helper T cells capable of activating B cells thus leading to antibody production, may also be induced. A variety of techniques may be used for analyzing the type of immune responses restored or induced by the compositions of the present invention, which are well described in the art; *e*.*g*., Coligan et al. Current Protocols in Immunology, John Wiley & Sons Inc. (1994).

When "an immunologically effective amount", "an anti-tumor effective amount", "an tumor-inhibiting effective amount", or "therapeutic amount" is indicated, the precise amount of the compositions of the present invention to be administered can be determined by a physician with consideration of individual differences in age, weight, tumor size, extent of infection or metastasis, and condition of the patient. Typically, in adoptive immunotherapy studies, activated antigen-specific T cells are administered approximately at 2 X 10⁷ to 2 X 10¹¹ cells to the patient. (See, *e*.*g*., U.S. Pat. No. 5,057,423). Particularly in the use of allogeneic or xenogeneic cells, lower numbers of cells, in the range of 10⁶/kilogram (10⁶-10¹¹ per patient) may be administered. In one embodiment of the present invention, T cells are administered approximately at 1 X 10⁸ cells to the patient. T cell compositions may be administered multiple times at dosages within these ranges. The activated T cells may be autologous or heterologous to the patient undergoing therapy. If desired, the treatment may also include administration of mitogens (*e*.*g*., PHA) or lymphokines, cytokines, and/or chemokines (*e*.*g*., GM-CSF, IL-4, IL-13, Flt3-L, RANTES, MIP1α, etc.) as described herein to enhance induction of the immune response.

In certain aspects of the present invention, the administered T cells maintain their polyclonality in vivo following administration for at least between 2 weeks and 1 year. In further embodiments, the administered T cells maintain polyclonality for 3, 4, 5, 6, 7, 8, 9, 10, 11, 12, 13, 14, 15, 16, 17, 18, 19, or 20 weeks following administration. In yet further embodiments, the administered T cells maintain polyclonality for at least 5, 5.5, 6, 6.5, 7, 7.5, 8, 8.5, 9, 9.5, 10,10.5, 11, 11.5, 12 months, or longer, following administration.

The administration of the subject pharmaceutical compositions may be carried out in any convenient manner, including by aerosol inhalation, injection, ingestion, transfusion, implantation or transplantation. The compositions may be administered to a patient subcutaneously, intradermally, intramuscularly, by intravenous (i.v.) injection, intratumorally, or intraperitoneally. Preferably, the T cell compositions are administered by i.v. injection. The compositions of activated T cells may be injected directly into a tumor or lymph node.

In yet another embodiment, the pharmaceutical composition can be delivered in a controlled release system. In one embodiment, a pump may be used (see Langer, 1990, Science 249:1527-1533; Sefton 1987, CRC Crit. Ref. Biomed. Eng. 14:201; Buchwald et al., 1980; Surgery 88:507; Saudek et al., 1989, N. Engl. J. Med. 321:574). In another embodiment, polymeric materials can be used (see Medical Applications of Controlled Release, 1974, Langer and Wise (eds.), CRC Pres., Boca Raton, Fla.; Controlled Drug Bioavailability, Drug Product Design and Performance, 1984, Smolen and Ball (eds.), Wiley, New York; Ranger and Peppas, 1983; J. Macromol. Sci. Rev. Macramol. Chem. 23:61; see also Levy et al., 1985, Science 228:190; During et al., 1989, Ann. Neurol. 25:351; Howard et al., 1989, J. Neurosurg. 71:105). In yet another embodiment, a controlled release system can be placed in proximity of the therapeutic target, thus requiring only a fraction of the systemic dose (see, e.g., Medical Applications of Controlled Release, 1984, Langer and Wise (eds.), CRC Pres., Boca Raton, Fla., vol. 2, pp. 115-138).

The T cell compositions may also be administered using any number of matrices. Matrices have been utilized for a number of years within the context of tissue engineering (see, *e*.*g*., Principles of Tissue Engineering (Lanza, Langer, and Chick (eds.)), 1997. The present invention utilizes such matrices within the novel context of acting as an artificial lymphoid organ to support, maintain, or modulate the immune system, typically through modulation of T cells. Accordingly, those matrix compositions and formulations which have demonstrated utility in tissue engineering may be used. Accordingly, the type of matrix that may be used is virtually limitless and may include both biological and synthetic matrices. In one particular example, the compositions and devices set forth by U.S. Patent Nos: 5,980,889; 5,913,998; 5,902,745; 5,843,069; 5,787,900; or 5,626,561 are utilized. Matrices comprise features commonly associated with being biocompatible when administered to a mammalian host. Matrices may be formed from both natural or synthetic materials. The matrices may be non-biodegradable in instances where it is desirable to leave permanent structures or removable structures in the body of an animal, such as an implant; or biodegradable. The matrices may take the form of sponges, implants, tubes, telfa pads, fibers, hollow fibers, lyophilized components, gels, powders, porous compositions, liposomes, cells, or nanoparticles. In addition, matrices can be designed to allow for sustained release of seeded cells or produced cytokine or other active agent. The matrix is flexible and elastic, and may be described as a semisolid scaffold that is permeable to substances such as inorganic salts, aqueous fluids and dissolved gaseous agents including oxygen.

A matrix is used herein as an example of a biocompatible substance. However, wherever the term matrix or matrices appears these terms should be read to include devices and other substances which allow for cellular retention or cellular traversal, are biocompatible, and are capable of allowing traversal of macromolecules either directly through the substance such that the substance itself is a semi-permeable membrane or used in conjunction with a particular semi-permeable substance.

### EXAMPLE 1

### T CELL STIMULATION

In certain experiments described herein, the process referred to as XCELLERATE I™ was utilized. In brief, in this process, the XCELLERATED T cells are manufactured from a peripheral blood mononuclear cell (PBMC) apheresis product After collection from the patient at the clinical site, the PBMC apheresis are washed and then incubated with "uncoated" DYNABEADS® M-450 Epoxy. During this time phagocytic cells such as monocytes ingest the beads. After the incubation, the cells and beads are processed over a MaxSep Magnetic Separator in order to remove the beads and any monocytic/phagocytic cells that are attached to the beads. Following this monocyte-depletion step, a volume containing a total of 5 x 10⁸ CD3⁺ T cells is taken and set-up with 1.5 x 10⁹ DYNABEADS® M-450 CD3/CD28 T Cell Expander to initiate the XCELLERATE™ process (approx. 3:1 beads to T cells). The mixture of cells and DYNABEADS^{®} M-450 CD3/CD28 T Cell Expander are then incubated at 37°C, 5% CO₂ for approximately 8 days to generate XCELLERATED T Cells for a first infusion. The remaining monocyte-depleted PBMC are cryopreserved until a second or further cell product expansion (approximately 21 days later) at which time they are thawed, washed and then a volume containing a total of 5 x 10⁸ CD3⁺ T cells is taken and set-up with 1.5 x 10⁹ DYNABEADS^{®} M-450 CD3/CD28 T Cell Expander to initiate the XCELLERATE Process for a second infusion. During the incubation period of ≈8 days at 37°C, 5% CO₂, the CD3⁺ T cells activate and expand. The anti-CD3 mAb (clone BC3; XR-CD3) is obtained from the Fred Hutchinson Cancer Research Center, Seattle, WA and the anti-CD28 mAb (clone B-T3; XR-CD28) is obtained from Diaclone (Besançon, France).

With a modified process referred to as XCELLERATE II™ the process described above was utilized with some modifications in which no separate monocyte depletion step was utilized and in certain processes the cells were frozen prior to initial contact with beads and further concentration and stimulation were performed. In one version of this process T cells were obtained from the circulating blood of a donor or patient by apheresis. Components of an apheresis product typically include lymphocytes, monocytes, granulocytes, B cells, other nucleated cells (white blood cells), red blood cells, and platelets. A typical apheresis product contains 1 - 2 x10¹⁰ nucleated cells. The cells are washed with calcium-free, magnesium-free phosphate buffered saline to remove plasma proteins and platelets. The washing step was performed by centrifuging the cells and removing the supernatant fluid, which is then replaced by PBS. The process was accomplished using a semi-automated "flow through" centrifuge (COBE 2991 System, Gambro BCT, Lakewood, CO). The cells are maintained in a closed system as they are processed.

The cells may be further processed by depleting the non-binding cells, including monocytes, (enriched for activated cells) and then continuing with the stimulation. Alternatively, the washed cells can be frozen, stored, and processed later, which is demonstrated herein to increase robustness of proliferation as well as depleting granulocytes. In one example, to freeze the cells, a 35 ml suspension of cells is placed in a 250 ml Cryocyte™ freezing bag (Baxter) along with 35 ml of the freezing solution. The 35 ml cell suspension typically contains 3.5x10⁹ to 5.0x10⁹ cells in PBS. An equal volume of freezing solution (20% DMSO and 8% human serum albumin in PBS) is added. The cells are at a final concentration of 50x10⁶ cells/ml. The Cryocyte bag may contain volumes in the range of 30 - 70 ml, and the cell concentration can range from 10 to 200x10⁶ cells/ml. Once the Cryocyte bag is filled with cells and freezing solution, the bag is placed in a controlled rate freezer and the cells are frozen at 1°C/minute down to-80°C. The frozen cells are then placed in a liquid nitrogen storage system until needed.

The cells are removed from the liquid nitrogen storage system and are thawed at 37°C. To remove DMSO, the thawed cells are then washed with calcium-free, magnesium-free PBS on the COBE 2991 System. The washed cells are then passed through an 80 micron mesh filter.

The thawed cells, approximately 0.5x10⁹ CD3⁺ cells, are placed in a plastic 1L Lifecell bag that contains 100 ml of calcium-free, magnesium-free PBS. The PBS contains 1% - 5% human serum. 1.5x10⁹ 3x28 beads (Dynabeads M-450 CD3/CD28 T Cell Expander) are also placed in the bag with the cells (3:1 DYNABEADS M-450 CD3/CD28 T Cell Expander:CD3⁺ T cells). The beads and cells are mixed at room temperature at 1 RPM (end-over-end rotation) for about 30 minutes. The bag containing the beads and cells is placed on the MaxSep Magnetic Separator (Nexell Therapeutics, Irvine, CA). Between the bag and the MaxSep, a plastic spacer (approximately 6 mm thick) is placed. (To increase the magnetic strength the spacer can be removed.) The beads and any cells attached to beads are retained on the magnet while the PBS and unbound cells are pumped away.

The 3x28 beads and concentrated cells bound to the beads are rinsed with cell culture media (1 liter containing X-Vivo 15, BioWhittaker; with 50 ml heat inactivated pooled human serum, 20 ml 1M Hepes, 10 ml 200 mM L-glutamine with or without about 100,000 I.U. IL-2) into a 3L Lifecell culture bag. After transferring the 3x28 beads and positively selected cells into the Lifecell bag, culture media is added until the bag contains 1000 ml. The bag containing the cells is placed in an incubator (37°C and 5% CO₂) and cells are allowed to expand, passaging the cells as necessary.

T cell activation and proliferation were measured by harvesting cells after 3 days and 8 days in culture. Activation of T cells was assessed by measuring cell size, the level of cell surface marker expression, particularly the expression of CD25 and CD 154 on day 3 of culture. On day 8 cells were allowed to flow under gravity (approx. 150 ml/min) over the MaxSep magnet to remove the magnetic particles and the cells were washed and concentrated using the COBE device noted above and resuspended in a balanced electrolyte solution suitable for intravenous administration, such as Plasma-Lyte A® (Baxter-Healthcare). Cells may also be frozen in appropriate freezing solution at this point.

As described, the XCBLLERATE I™ refers to conditions similar to that above, except that stimulation and concentration were not performed and monocyte depletion was performed prior to stimulation.

Monocyte-depleted PBMC from 4 donors were stimulated with 3x28 coupled beads (Dynabeads M-450 CD3/CD28 T Cell Expander). The concentration of IL₋2, IL-4, TNF-α, and IFN-γ in the supernatant was determined by ELISA. Concentrations of IL-4, TNF-α, and IFN-γ, were also measured following reseeding of the cells with new Dynabeads M-450 CD3/CD28 T Cell Expander on day 12 (re-stimulation).

As shown in Table 1, Table 2, and Table 3, concentrations of IFN-γ, IL-4, and TNF-α, were measured by ELISA on various days during XCELLERATE™ and Re-stimulation.

**TABLE 1: PRODUCTION OF INTERFERON-γ BY T CELLS ON DAY 3 OF THE XCELLERATE™ PROCESS AND ON DAY 3 OF RE-STIMULATION OF XCELLERATE™ ACTIVATED T CELLS**

| | **XCELLERATE™ Process Day 2 [IFN-γ] ng/mL** | **Re-stimulation Day 2 [IFN-γ] ng/ml** |
|---|---|---|
| **Average** | 13.61 | 31.59 |
| **Range** | 7.99-27.11 | 10.8-95.5 |
| **Standard Dev.** | 5.64 | 22.98 |
| **Median** | 11.95 | 26.4 |
| **N** | 24 | 24 |

| | | |
|---|---|---|
| Phagocyte-depleted PMBC from 3 donors were stimulated with anti-CD3 & anti-CD28 coupled to Dynabeads M-450 Epoxy (Dynabeads CD3/CD28 T Cell Expander) (XCELLERATE™). The concentration of IFN-γ in the supernatant was determined on Day 2 by ELISA. On Day 12, cells were re-seeded with new anti-CD3 & anti-CD28 coupled Dynabeads M-450 Epoxy (re-stimulation) and the concentration of IFN-γ determined 2 days later. | | |

**TABLE 2: PRODUCTION OF IL-4 BY T CELLS ON DAY 2 OF THE XCELLERATE™ PROCESS AND ON DAY 2 OF RE-STIMULATION OF XCELLERATE™ ACTIVATED T CELLS**

| | **XCELLERATE™ Process Day 2 [IL-4] pg/ml** | **Re-stimulation Day 2 [IL-4] pg/ml** |
|---|---|---|
| **Average** | 310 | 274 |
| **Range** | 170-460 | 50-500 |
| **Standard Dev.** | 143 | 224 |
| **Median** | 297 | 268 |
| **N** | 3 | 3 |

| | | |
|---|---|---|
| Phagocyte-depleted PMBC from 3 donors were stimulated with anti-CD3 & anti-CD28 coupled to Dynabeads M-450 Epoxy (XCELLERATE™). The concentration of IL-4 in the supernatant was determined on days 2 & 4 by ELISA. On Day 12, cells were re-seeded with new anti-CD3 & anti-CD28 coupled Dynabeads M-450 Epoxy (re-stimutation) and the concentration of IL-4 determined 2 days later. | | |

**TABLE 3: PRODUCTTON OF TNF-α BY T CELLS ON DAY 2 & DAY 4 OF THE XCELLERATE™ PROCESS AND ON DAY 2 & DAY 4 OF RE-STIMULATION OF XCELLERATE™ ACTIVATED T CELLS**

| | **Day 2** | | **Day 4** | |
|---|---|---|---|---|
| | **XCELLER ATE™ [TNF-α] ng/mL** | **Re-stimulation [TNF-α] ng/mL** | **XCELLER ATE™ [TNF-α] ng/mL** | **Re-stimulation [TNF-α] ng/mL** |
| **Average** | 1.710 | 0.594 | 1.635 | 0.252 |
| **Range** | 1.11-2.81 | 0.299 - 0.782 | 1.09-2.5 | 0.21-0.288 |
| **Standard Dev.** | 0.762 | 0.211 | 0.534 | 0.036 |
| **Median** | 1.460 | 0.647 | 1.55 | 0.255 |
| **N** | 4 | 4 | 4 | 4 |

| | | | | |
|---|---|---|---|---|
| Phagocyte-depleted PMBC from 4 donors were stimulated with anti-CD3 & anti-CD28 coupled to Dynabeads M-450 Epoxy. The concentration of TNF-α, in the supernatant was determined on the days 2 & 4 by ELISA. On Day 12, cells were re-seeded with new anti-CD3 & anti-CD28 coupled Dynabeads M-450 Epoxy (re-stimulation) and the concentration of TNF-α determined 2 & 4 days later. | | | | |

Expression levels of CDw137 (41BB), CD154 (CD40L), and CD25 on Xcellerated T cells were analyzed by flow cytometry, and the mean fluorescence plotted. Expression levels of CDw137 (41BB) increase and peak at day 4 and then decrease gradually. Following re-stimulations, expression of CDw137 increased rapidly. Expression of CD154 increases gradually until about day 7 and then decreases. Following re-stimulation, however, levels of C154 increase rapidly and to much higher levels than during the initial stimulation. Levels of CD25 increased until about day 3 and then decreased gradually until day 8 (the last time point analyzed).

### EXAMPLE 2

### SPECTRA TYPE ANALYSIS OF T CELLS

This example describes the use of spectratype analysis to determine the clonality of the expressed TCRs in T cell populations before and after stimulation using the XCELLERATE^{™} method. Described herein is the analysis of rearranged Vβ genes. The skilled artisan will readily recognize that the Vα, Vγ, and Vδ TCR genes may be analyzed in a similar manner.

Spectratype analysis was carried out essentially as described in U.S. Patent No. 5,837,447, and C. Ferrand, et al (C. Ferrand, E. Robinet, Emmanuel Contassot, J-M Certoux, Annick Lim, P. Herve, and P. Tiberghien. Human Gene Therapy 11: 1151-1164, 2000). Briefly, starting cell suspensions were from PBMCs, cell lines, PBMC depleted of CD8+ cells, and/or XCELLERATED T cells. Total RNA was isolated using Trizol (Gibco-BRL) and 2 ug were reverse transcribed with random hexamers (Pharmacia Biotech) in a standard cDNA synthesis reaction.

Each TCR BV segment was amplified with 1 of the 24 TCR BV subfamily-specific primers and a Cβ primer recognizing the two constant regions Cβ1 and Cβ2 of the β chain of the TCR, as previously described (Puisieux, et al., 1994, J. Immunol. 153, 2807-2818; Pannetier et al., 1995, Immunol. Today 16:176-181). The Cβ primer was coupled with a 6-Fam fluorescent dye (Gibco-BRL). For the quantitative analysis, cDNAs were coamplified with an internal standard (PTZ-δCD3 plasmid).

Aliquots of the cDNA synthesis reaction were amplified in a thermocycler in a 25-µl reaction with 1 of the 24 TCRBV oligonucleotides and the nonlabled Cβ primer.

*PCR amplification for TCRBV transcript size pattern.* Aliquots of the cDNA synthesis reaction (corresponding to 85 ng of total RNA) were amplified in a thermocycler (PTC-200; MJ Research, Watertown, MA) in a 25-µl reaction with 1 of the 24 TCRBV oligonucleotides and the nonlabeled C_{β} primer. Each reaction contained 1 x *Taq* polymerase buffer (Promega, Charbonniere, France), 1.5 m*M* MgCl₂, a 0.2 µ*M* concentration of each dNTP, a 0.5 µ*M* concentration of each primer, and 0.5 U of *Taq* polymerase (Promega). A PCR was performed at saturation, using the following program: predenaturation for 3 min at 94°C; 40 cycles of denaturation (25 sec at 94°C), annealing (45 sec at 60°C), and polymesization (45 sec at 72°C); followed by a final extension for 5 min at 72°C. Electrophoresis in 2% agarose was performed for some TCRBV/C_{β} PCR products and the negative control (absence of cDNA) included in the assay, in order to check amplification and possible contamination. Two microliters of each of the 24 TCRBV/C_{β}-40 cycle PCR products was subjected to two cycles of elongation (runoff) under the same conditions, except that the C_{β} fluorescent primer was at a final concentration of 0.1 µ*M* in 10 µl.

### Quantification of TCRBV subfamily representation in cell populations

*Competitive δCD3 PCR.* For each sample, the synthesized cDNAs were amplified by adding serially diluted defined amounts of DNA plasmid (4 bp-deleted δCD3 chain) ranging from 10¹¹ to 10⁷ copies of competitor (Garderet et al., 1998). The optimal titration point was defined as the concentration of standard at which PCR products yielded signals of comparable intensity for standard and native cDNA. Briefly, δCD3 PCR was performed in a 25 µl reaction using 1X *Taq* polymerase buffer (Promega), 1.5 m*M* MgCl₂, a 0.2 µ*M* concentration of each dNTP, a 0.5 µ*M* concentration of each primer, and 0.5 U of *Taq* polymerase (Promega). The PCR was performed at saturation, using the following program: predenaturation for 3 min at 94°C; 40 cycles of denaturation (1 min at 94°C) annealing (1 min at 60°C), and polymerization (45 sec at 72°C); followed by a final extension for 5 min at 72°C. Two microliters of the first PCR was stained during two cycles of elongation under the same conditions, except that the 3' δCD3 fluorescent primer was at a final concentration of 0.1 µ*M* in a volume of 10 µl. Fluorescent PCR products were separated on a denaturant 6% acrylamide gel and analyzed on an automated DNA sequencer with Genescan version 1.2.1 (Applied Biosystems, Foster City, CA) analysis software.

*Quantitative TCRBV*/*C_{β} PCR.* To quantify the TCRBV subfamily representation in a full repertoire, the 24 TCRBV/C_{β} reactions (15 µl) were performed during the linear phase of the PCR (26-28 cycles) from cDNA (corresponding to 5 X 10⁷ copies of δCD3 RNA) under conditions similar to those described for the 40 amplification cycles, except for the use of a C_{β} fluorescent primer at a concentration of 0.1 µ*M* for each TCRBV subfamily primer. For the TCRBV representation in the full repertoire, the relative percentage of each TCRBV subfamily was calculated by dividing the sum of all peaks of a TCRBV subfamily by the sum of all TCRBV subfamilies. Because the initial number of δCD3 copies was equivalent in all TCRBV PCRs, all samples were comparable to each other.

*Electrophoresis and CDR3 fragment size analysis.* The reactions of both 40-cycle and 26- to 28-cycle PCR amplifications were mixed with an equal volume (10 or 15 µl, respectively) of 20 m*M* EDTA-deionized formamide, Rox-1000 size standard was used as molecular weight marker (Applied Biosystems). A 2.5-µl volume of the mix was loaded on a 24-cm 6% acrylamide sequencing gel and analyzed on an automated 373A DNA sequencer (Applied Biosystems) for size and fluorescence intensity determination with Immunoscope software.

The polymerase chain reaction (PCR) product lengths using this technique reflect the CDR3 lengths of the input TCR RNA, being dependent upon joining (J) and diversity (D) gene segment usage along with the balance of exonuclease activity and N nucleotide addition by terminal transferase at the junctional regions. Peaks corresponding to in-frame transcripts are detected.. The appearance of a dominant peak suggests the presence of an oligoclonal or clonal T-cell population, while the absence of peaks or entire subfamily spectratypes suggests the absence of T cells of the given CDR3 length or V_{β} subfamily, respectively, or the absence of TCR transcripts in T cells with productive TCR gene rearrangements.

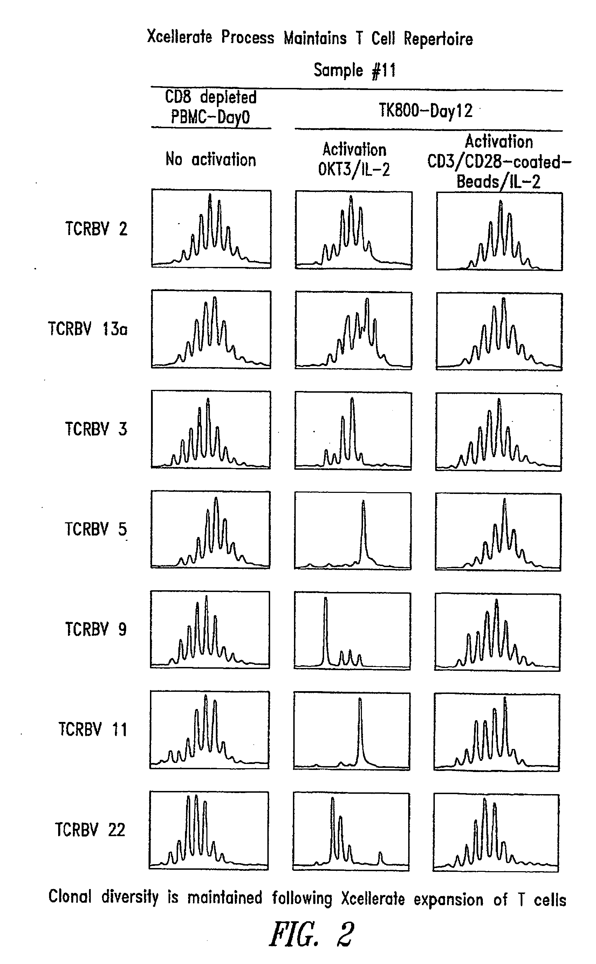

As shown in Figure 2, T cell repertoire was maintained using the XCELLERATET™ process as compared to the skewing of the repertoire seen when T cells are activated using OKT3/IL-2. T cells from a B-CLL patient were analyzed before and after the XCELLERATE™ activation process. As shown in Figure 3 (in particular, panels in the 4^{th} row, V_{β} 4, 9, 15, and 22) patients with B-CLL show a skewed T cell repertoire, *i.e.,* show a reduced polyclonality for T cells expressing numerous Vβ family genes, including Vβ 4, 9, 11, 13, 14, 15, and 22. Spectratype analysis of T cells on day 12 of the XCELLERATE™ process shows a restoration of the polyclonality of these T cells.

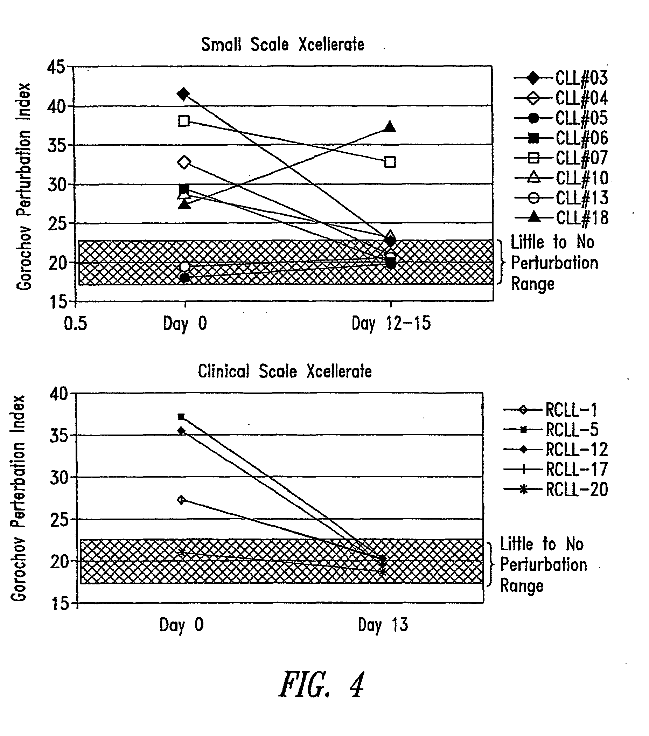

Figure 4 uses the Gorochov analysis (G. Gorochov, et al. Nat.Med, 4: 215-221, 1998.) to ascribe a value summing the total level of repertoire "perturbation" for each donor prior to and post-XCELLERATE™ expansion. Figure 4a reflects values obtained from 8 different B-CLL donors prior to and post small-scale XCELLERATE™ expansions, while Figure 4b reflects values obtained for 5 different donors prior to and post-clinical scale XCELLERATE™ expansions. With the exception of one donor, where an already skewed repertoire became more skewed, all other donors that had initial skewing gravitated towards normalization (Gaussian distribution). Eight of the 13 samples analyzed went from high levels of repertoire perturbation back to normal levels. One of the 13 samples exhibited a reduction in perturbation, but not to levels considered normal, and 3 donor samples that were not skewed to start with retained normal distribution throughout the expansion process.

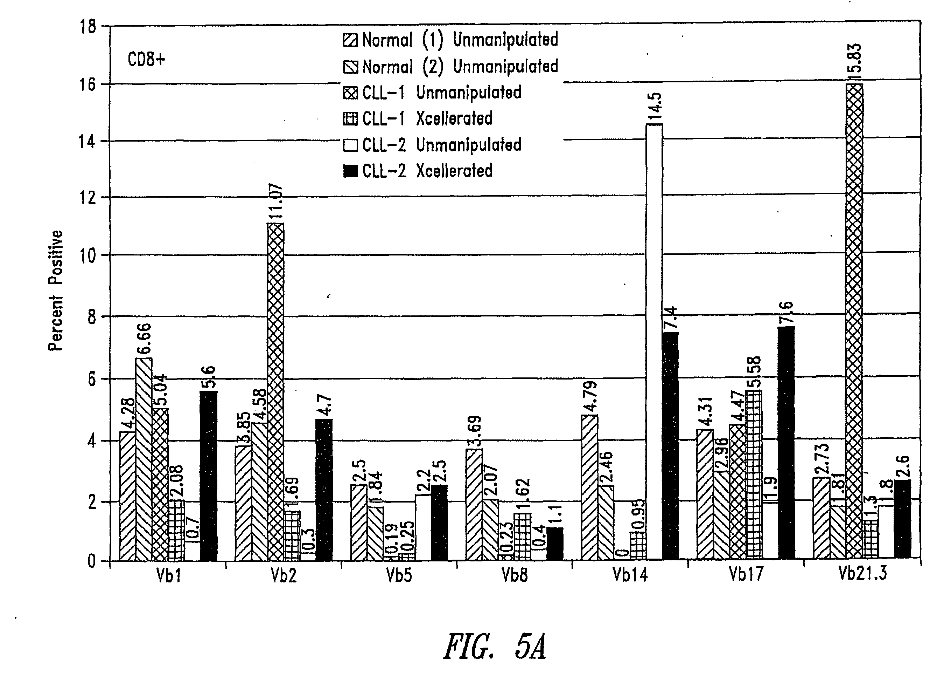

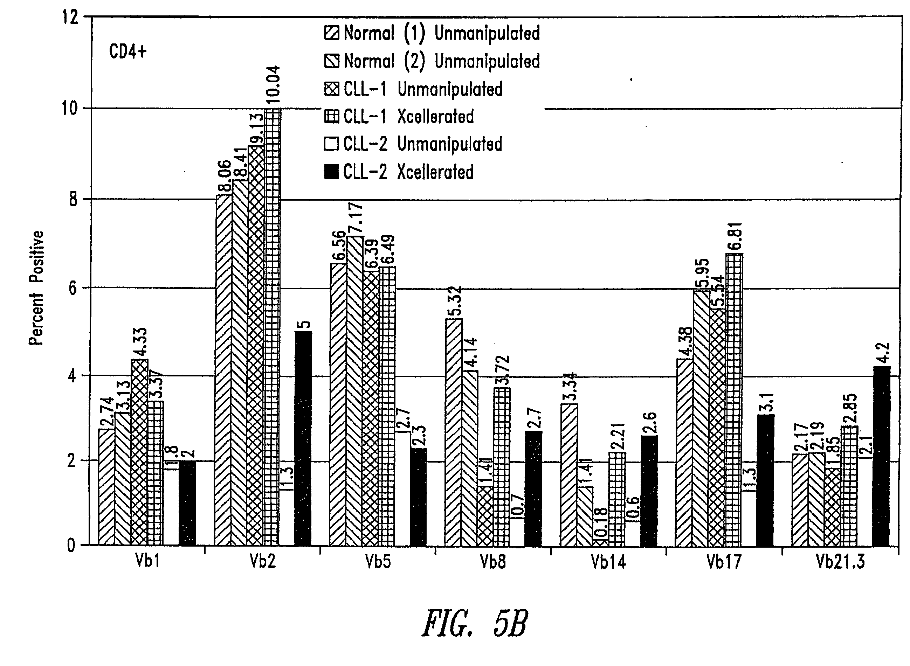

TCR Vβ usage was also examined by analyzing surface expression of various TCR Vβ families by flow cytometry using standard techniques employing antibodies with specificities for members of distinct Vβ families.. As shown in Figure 5a and 5b, the percentage of CD4 T cells and CD8 T cells that expressed representative TCR Vβ families proteins on their surface (Vβ's 1, 2, 5, 8, 14, 17, and 21.3) was determined. T cells isolated from 2 normal donors and T cells isolated from 2 CLL donors were analyzed. In the case of the B-CLL sample, the plots reflect before and after XCELLERATE™ patterns. From these data, it is apparent that each of the B-CLL samples demonstrates both over- and under-representation of particular Vβ families, particularly among the CD8 populations. For example, prior to activation and expansion, amongst the CD8 T cell population, CLL donor 1 has very high percentages of Vβ2 and Vβ21.3 expressing T cells, while CLL donor 2 has a high percentage of Vβ14 expressing T cells. In contrast, these same two donors show extremely low percentages of Vβ5, 8, 14 expressers for donor 1 and Vβ1, 2, 8 expressers for donor 2. Similar to observations in the spectratype studies, after expansion via the XCELLERATE™ process, these percentages trend towards more normal levels, with the percentage of over-expressers coming down, and the percentage of underrepresented Vβ's rising.

In order to evaluate the frequency of T cells with specificity towards the leukemic B cells, interferon-gamma (IFNγ) ELISPOT analysis was performed by mixing XCELLERATED^{™} T Cells with autologous leukemic B cell targets. As shown in Table 4, tumor-specific T cells were detectable in the range of 1:167 to 1:2,500. Frequencies pre-XCELLERATE^{™} were < 1:10,000 (limit of sensitivity), suggesting that tumor-specific T cells had been selectively amplified in number, or, more likely, tumor-specific T cells were anergic prior to activation and expansion, and the XCELLERATE^{™} process restored responsiveness. The frequency of tumor reactive T cells reported reflects the subtraction of background frequency of IFNy positive cells in the absence of CLL stimulatory cells.

**TABLE 4: FREQUENCY OF TUMOR-REACTTVE T CELLS FOLLOWING XCELLERATE^{™} EXPANSION**

| | | Frequency of Tumor-Reactive T Cells Measured by ELISPOT | |
|---|---|---|---|
| Experiment | Scale | Donor | (IFNγ) |
| CLL-3 | Small-scale | OHSU-10 | 1:256 |
| CLL-4 | Small-scale | OHSU-11 | 1:556 |
| CLL-5 | Small-scale | OHSU-12 | 1:333 |
| CLL-6 | Small-scale | OHSU-17 | 1:681 |
| CLL-7 | Small-scale | OH-CL-101B | 1:284 |
| CLL-8 | Small-scale | OH-CL-103B | 1:850 |
| CLL-18 | Small-scale | RCLL-1 | 1:1667 |
| CLL-20 | Small-scale | OH-CL-105L | 1:200 |
| CLL-23 | Small-scale | OHSU-16 | 1:2500 |
| CLL-30 | Small-scale | RCLL-7 | 1:1111 |
| CLL-31 | Small-scale | RCLL-14 | 1:1000 |
| CLL-32 | Small-scale | RCLL-14 | 1:909 |
| CLL-33 | Small-scale | RCLL-8 | 1:1111 |
| CLL-34 | Small-scale | RCLL-7 | 1:555 |
| CPDCLL-12 | Wave | RCLL-8 | 1:167 |
| CPDCLL-13 | Wave | RCLL-7 | 1:833 |
| **MEAN (N=16)** | | | 1:813 |

| | | | |
|---|---|---|---|
| Table 4. 13/16 Xcellerated T Cells from 14 small-scale expansions and 2 large-scale expansions were evaluated for frequency of anti-tumor-specific T cells by ELISPOT. Tissues were obtained from 13 different donors. | | | |

Thus, as shown herein, not only can reduced TCR expression and thus responsiveness to antigen be restored, but the breadth of the immune response can be widened by ex-vivo Xcelleration of an individual's T cells. The XCELLERATE™ process can be used to maintain or restore polyclonality in T cells. Xcellerated T cells with increased polyclonality of the present invention can be used as a prophylactic measure or for treatment of existing ailments, such as B-CLL. Activated and expanded T cells generated using this process can thus be used to restore immune responsiveness in immunocompromised individuals.

### EXAMPLE 3

### XCELLERATE PROCESS IMPROVES LYMPHOCYTE RECOVERY IN TRANSPLANTED MYELOMA PATIENTS

This example describes data from a preliminary clinical trial in a patient with multiple myeloma indicating that XCELLERATED T cells improve the recovery in transplanted myeloma patients.

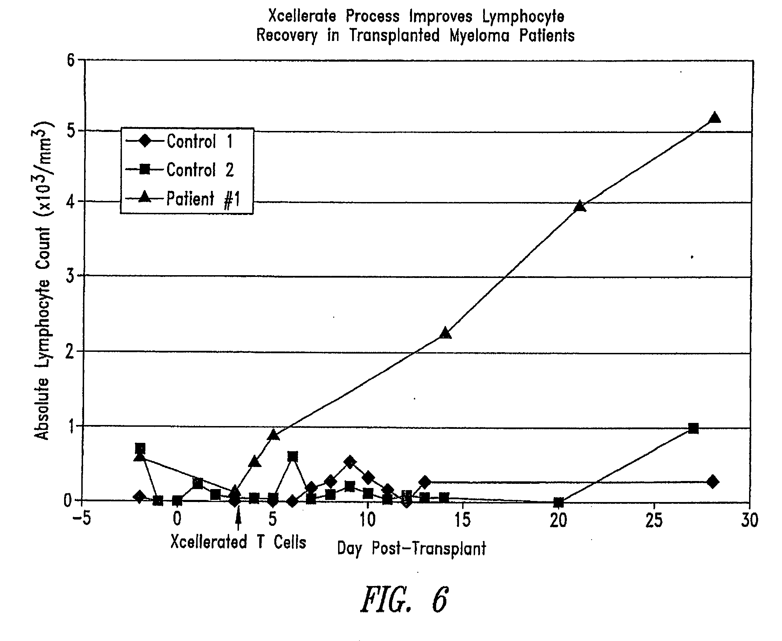

The XCELLERATE II process was carried out essentially as described in Example 1 on leukapheresed cells collected from the patient following registration in the clinical trial and prior to stem cell collection. XCELLERATED T cells were infused on day +3 following stem cell infusion. As shown in Figure 6, the XCELLERATE^{™} process improves lymphocyte recovery in a transplanted myeloma patient, Additionally, both CD4 and CD8 T cells increased after XCELLERATED T cell infusion.

Thus, this clinical data shows that XCELLERATED T cells improve the recovery in transplanted myeloma patients and support the notion that the T cell compositions described herein can be infused into donors to provide broad and potent immune protection.

## Claims

1. An ex vivo method for normalising a population of T cells obtained from an individual with respect to the repertoire of at least one Vβ family gene expressed, wherein the at least one Vβ family gene is selected from the group consisting of Vβ 4, 9, 11, 13, 14, 15 and 22, said method comprising:
(a) providing a population of cells from an individual wherein at least a portion thereof comprises T cells;
(b) exposing the population of cells to a first and a second agent, wherein the first agent comprises an anti-CD3 antibody, or antigen-binding fragment thereof, and the second agent comprises an anti-CD28 antibody, or antigen-binding fragment thereof; wherein said first and second agents are attached to a surface; and wherein the exposure of said T cells to said first or second agent is for a time sufficient to normalise with respect to expression of at least one Vβ family gene.

2. The method of claim 1 wherein said first and second agents are attached to said surface by covalent attachment, by direct attachment or by indirect attachment.

3. A method of formulating the normalised T cell population obtained by the method of claim 1 or 2, wherein said normalised T cell population is formulated with one or more pharmaceutically or physiologically acceptable carriers, diluents or excipients.

## Patentansprüche

1. Ex vivo Verfahren zum Normalisieren einer Population von T-Zellen, die von einer Person hinsichtlich des Repertoires wenigstens eines exprimierten Vβ-Familiengens erhalten worden ist, wobei das wenigstens eine Vβ-Familiengen ausgewählt ist aus der Gruppe bestehend aus Vβ 4, 9, 11, 13, 14, 15 und 22, wobei das genannte Verfahren Folgendes umfasst:
(a) Bereitstellen einer Population aus Zellen von einer Person, wobei wenigstens ein Teil davon T-Zellen umfasst;
(b) Aussetzen der Population von Zellen an ein erstes und ein zweites Mittel, wobei das erste Mittel einen Anti-CD3-Antikörper oder ein antigenbindendes Fragment davon umfasst und das zweite Mittel einen Anti-CD28-Antikörper oder ein antigenbindendes Fragment davon umfasst; wobei die genannten ersten und zweiten Mittel an einer Oberfläche befestigt sind; und wobei das Aussetzen der genannten T-Zellen an das genannte erste oder zweite Mittel über einen Zeitraum stattfindet, der zum Normalisieren hinsichtlich der Expression des wenigstens einen Vβ-Familiengens ausreichend ist.

2. Verfahren nach Anspruch 1, wobei die genannten ersten und zweiten Mittel durch kovalente Befestigung, durch direkte Befestigung oder durch indirekte Befestigung an der genannten Oberfläche befestigt sind.

3. Verfahren zur Formulierung der durch das Verfahren nach Anspruch 1 oder 2 erhaltenen normalisierten T-Zellrepertoires, wobei der genannte normalisierte T-Zellrepertoire mit einem oder mehreren pharmazeutisch oder physiologisch akzeptablen Trägern, Verdünnungsmitteln oder Arzneistoffträgern formuliert ist.

## Revendications

1. Procédé ex vivo de normalisation d'une population de cellules T obtenue à partir d'un individu par rapport au répertoire d'au moins un gène de la famille V5 exprimé, le au moins un gène de la famille V5 étant choisi dans le groupe consistant en V5 4, 9, 11, 13, 14, 15 et 22, ledit procédé comprenant :
(a) se procurer une population de cellules à partir d'un individu, au moins une partie de celle-ci comprenant des cellules T ;
(b) exposer la population de cellules à un premier et à un second agent, le premier agent comprenant un anticorps anti-CD3, ou un fragment de liaison à un antigène de celui-ci, et le second agent comprenant un anticorps anti-CD28, ou un fragment de liaison à un antigène de celui-ci ; lesdits premier et second agents étant fixés à une surface ; et l'exposition desdites cellules T audit premier ou second agent se faisant pendant un temps suffisant pour normaliser par rapport à une expression d'au moins un gène de la famille V5.

2. Procédé selon la revendication 1, dans lequel lesdits premier et second agents sont fixés à ladite surface par fixation covalente, par fixation directe ou par fixation indirecte.

3. Procédé de formulation de la population de cellules T normalisée obtenue par le procédé de l'une des revendications 1 ou 2, dans lequel ladite population de cellules T normalisée est formulée avec un ou plusieurs supports, diluants ou excipients pharmaceutiquement ou physiologiquement acceptables.
